# EUROPEAN PATENT APPLICATION

(11) **EP 4 600 264 A1**
(43) Date of publication of application: **13.08.2025**
(21) Application number: 24156573.8
(22) Date of filing: 08.02.2024
(51) Int. Cl.: C07K 14/54, A61K 39/00

(54) **ENGINEERED TYPE 2 CYTOKINE SIGNALLING TO IMPROVE CAR-T CELL EFFECTOR FUNCTIONS**

(71) Applicant: Ecole Polytechnique Fédérale de Lausanne (EPFL), 1015 Lausanne (CH)
(72) Inventor: TANG, Li, 1112 Echichens (CH); ZHAO, Yang, Menlo Park, 94025 (US); ENBAR, Tom, 1006 Lausanne (CH); FENG, Bing, 1024 Ecublens (CH)
(74) Representative: KATZAROV S.A.

(57) **Abstract**

The invention relates to chimeric antigen receptor constructs and their use for treatments and therapies. Further disclosed are methods of treating and/or preventing a disease, such as e.g. a cancer, an infectious disease, an inflammatory or inflammation-induced disease, a chronic disease or an autoimmune disease.

## Description

### FIELD OF THE INVENTION

The invention relates to chimeric antigen receptor constructs and their use for treatments and therapies. Further disclosed are methods of treating and/or preventing a disease, such as e.g. a cancer, an infectious disease, an inflammatory or inflammation-induced disease, a chronic disease or an autoimmune disease.

### BACKGROUND OF THE INVENTION

Despite its success in treating hematologic malignancies, CAR-T cell therapy remains largely ineffective against solid tumors due to several obstacles that limit persistence and activity of CAR-T cells in the TME¹. These obstacles including T cell dysfunction, the heterogenous expression of tumor antigens, and insufficient tumor infiltration of T cells result in tumor progression beyond control by the CAR-T cells^{2,3}.

To improve therapeutic efficacy against solid cancers, the fourth generation CAR-T cells have been engineered to express stimulatory cytokines, which are known as T cells redirected for universal cytokine killing (TRUCKs) or armored CARs. These CAR-T cells armored with cytokines were applied to improve the persistence of infused CAR-T cells or stimulate endogenous immune cells. For example, CAR-T cells are modified T cells to co-express the CAR molecule and T cell stimulating cytokines such as IL-7, and IL-15 that improves CAR-T cell intrinsic activity without modulating neighboring endogenous immune populations^{4,5}. In addition, CAR-T cells were also engineered to express stimulatory cytokines such as IL-12 or IL-18 to activate both CAR-T cells and local immune cells⁶⁻⁸.

To date, the design of armored CAR-T cells was largely explored in type 1 cytokines but not in type 2 cytokines that can also assist antitumor immunity.

Strategies that overcome one or more of these obstacles provide the opportunities to improve the response rate of CAR-T cells against cancer, in particular solid tumors.

### SUMMARY OF THE INVENTION

The present invention provides a chimeric antigen receptor polypeptide comprising:
- at least one antigen binding domain,
- a transmembrane domain,
- at least one costimulatory domain,
- at least one activating domain, and
- at least one interleukin 4 (IL-4) molecule, a variant or a functional fragment thereof.

Further provided is an isolated polynucleotide encoding the chimeric antigen receptor (CAR) of the invention.

Also provided is a vector comprising an isolated polynucleotide of the invention.

Also provided is a host cell, preferably an immune cell, comprising i) chimeric antigen receptor polypeptide, an isolated polynucleotide, and/or a vector of the invention, or ii) expressing a chimeric antigen receptor according to the invention.

Also provided is a pharmaceutical composition comprising i) a chimeric antigen receptor polypeptide of the invention, ii) an isolated polynucleotide of the invention, iii) a vector of the invention, or iv) an immune cell of invention, and pharmaceutically acceptable carrier, diluent and/or excipient.

Further provided is a method of treating a cancer, an infectious disease, an inflammatory or inflammation-induced disease, a chronic disease or an autoimmune disease comprising administering i) a chimeric antigen receptor polypeptide of the invention, ii) an isolated polynucleotide of the invention, iii) a vector of the invention, iv) an immune cell of the invention, or v) a pharmaceutical composition of the invention, to a subject in need thereof.

### DESCRIPTION OF THE FIGURES

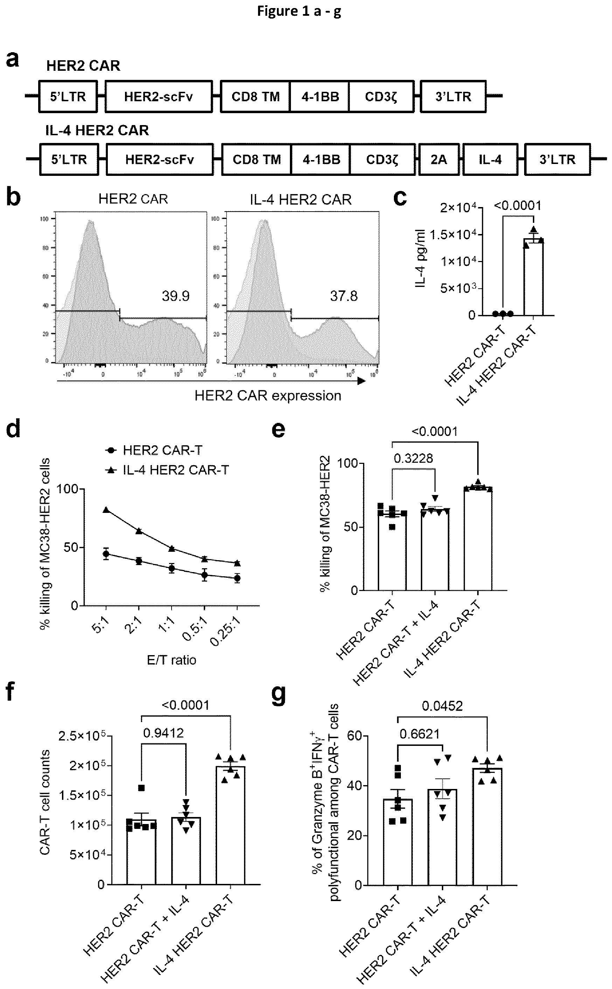

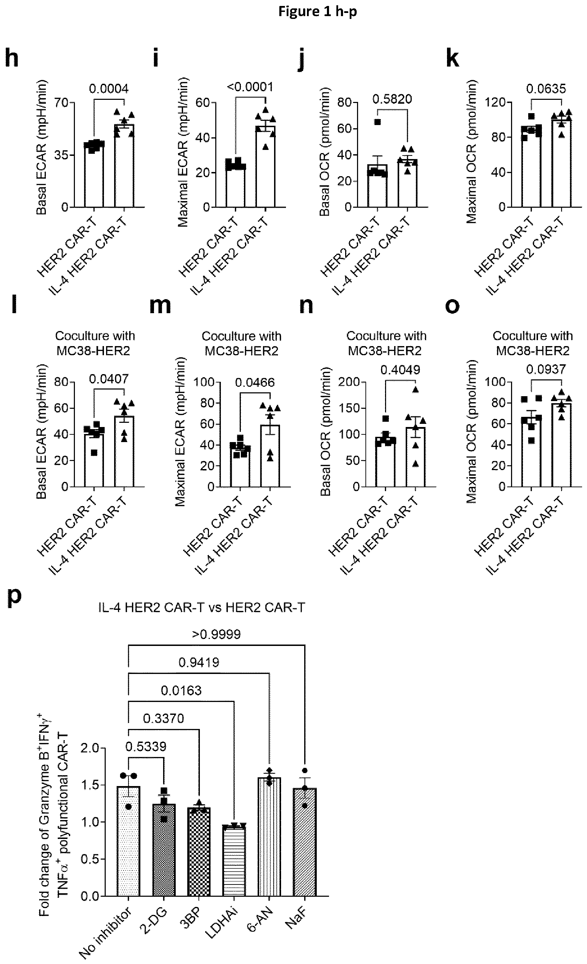

**Figure 1****. HER2-specific CAR-T cells co-expressing IL-4 (IL-4 HER2 CAR T) promotes anti-tumor activity and enhances polyfunction of CAR-T cells in a LHDA dependent glycolysis.** a, Schematic representations of HER2-directed second generation CAR (HER2 CAR), and HER2-directed second generation CAR modified to express murine IL-4 following a 2A element (HER2 CAR-IL-4). b, Transduction with HER2 CAR or HER2 CAR-IL-4 construct was conducted by retroviral vectors. The expression levels of CAR were analyzed by flow cytometry. The numbers in histograms represent the percentages of HER2 CAR positively stained cells. Similar results were obtained from five independent experiments. c, IL-4 HER2 CAR-T or HER2 CAR-T cells were cultured for 2 days. The culture supernatants were examined for the concentration of IL-4 by enzyme-linked immunosorbent assay (ELISA). d, Cytotoxicity assay using MC38-HER2 (HER2-expressing MC38 colon cancer cells) as targets. Target cells were mixed with HER2 CAR T or IL-4 HER2 CAR T cells at the indicated E/T ratios. e-g, IL-4 HER2 CAR-T cells or HER2 CAR-T cells in the absence or presence of mouse IL-4 (20 ng/ml) were cocultured with MC38-HER2 cells at the E:T ratio of 0.5:1 for 48 h. Shown are percentage of lysis of MC38-HER2 cells (e), viable CAR-T cell counts (f), and frequencies of granzyme B⁺IFNγ⁺ polyfunctional CAR-T cells (g). h-k, IL-4 HER2 CAR-T or HER2 CAR-T cells in culture were isolated for a seahorse assay. h,i, Average basal (h) and maximal (i) OCR. j,k, Average basal (j) and maximal (k) ECAR. l-o, IL-4 HER2 CAR-T or HER2 CAR-T cells were cocultured with MC38-HER2 cells at the E:T ratio of 5:1 for 18 h. CAR-T cells were isolated for a seahorse assay. l,m, Average basal (1) and maximal (m) OCR. n,o, Average basal (n) and maximal (o) ECAR. p, HER2 CAR-T or IL-4 HER2 CAR-T cells were cocultured with MC38-HER2 cells in the presence of indicated inhibitors. Shown are relative frequencies of granzyme B⁺IFNγ⁺TNFα⁺ polyfunctional CAR-T cells (IL-4 HER2 CAR-T vs HER2 CAR-T). All data represent the mean ± s.e.m. and are analyzed by unpaired Student's t-test (c, d, h-o) or ANOVA with Tukey's multiple-comparisons test (e-g, p).

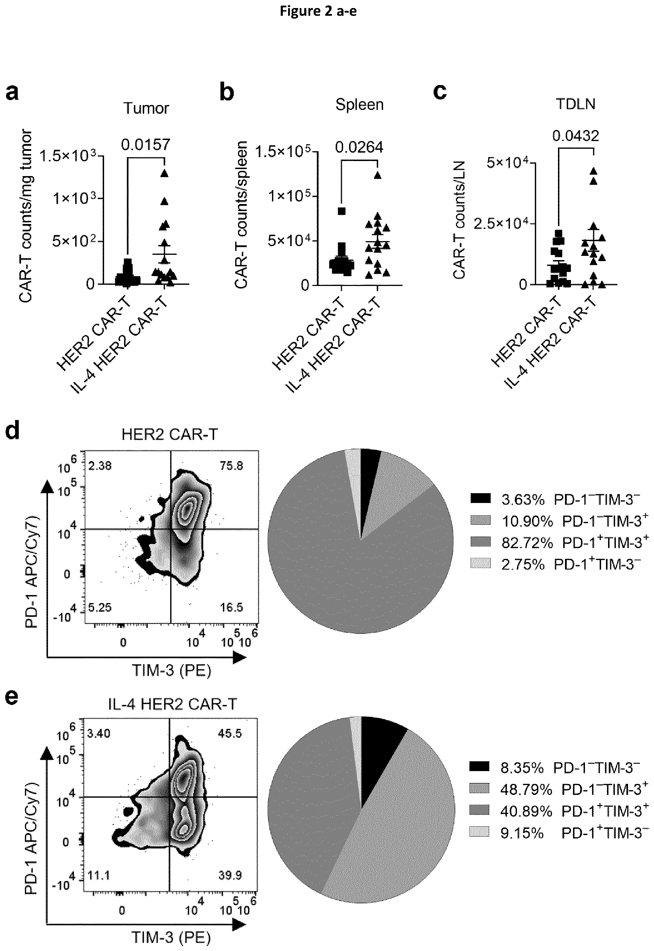

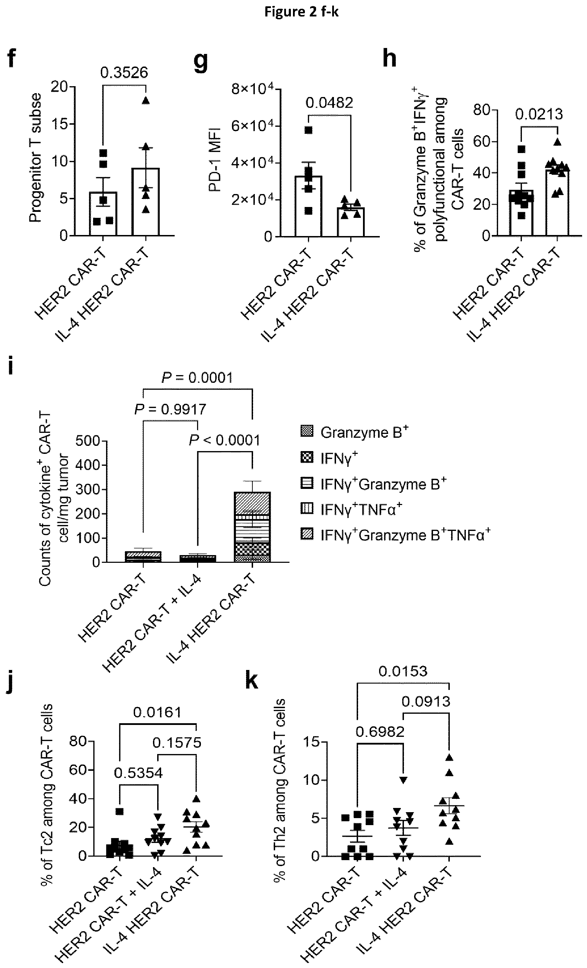

**Figure 2****. IL-4 HER2 CAR-T cells resist exhaustion and enrich type 2 T cell subsets in tumors.** C57BL/6 mice were inoculated with MC38-HER2 tumor cells (1 × 10⁶, s.c.), sublethally lymphodepleted by irradiation on day 5, and received i.v. adoptive transfer of IL-4 HER2 CAR-T cells (3 × 10⁶), or HER2 CAR-T cells (3 × 10⁶) in the presence or absence of i.v. administered IL-4 (1 µg) on day 6. On day 14, mice were killed and indicated tissues were processed. a-c, Counts of viable HER2 CAR-T cells in tumor (a), spleen (b), and tumor draining lymph node (TDLN) (c). d. Representative flow cytometry plots and average composition showing the frequencies of four subpopulation among HER2 CAR-T cells gated-based on PD-1and TIM-3 expression. e. Representative flow cytometry plots and average composition showing the frequencies of four subpopulation among IL-4 HER2 CAR-T cells gated-based on PD-1and TIM-3 expression. f, Frequencies of progenitor exhausted CAR-T cells. g, MFI of PD-1 in CAR-T cells in tumors. h, Representative flow cytometry plots and bar graph showing the frequencies of granzyme B+IFNγ⁺TNFα⁺ polyfunctional CAR-T cells in tumors. i, Counts of viable IFNγ⁺, granzyme B⁺, and polyfunctional HER2 CAR-T cells in tumors. j, Frequencies of Tc2 among CAR-T cells in tumors. k, Frequencies of Th2 among CAR-T cells in tumors. All data represent the mean ± s.e.m. and are analyzed by unpaired Student's t-test (a-c, f-h), ANOVA with Tukey's multiple-comparisons test (i-k).

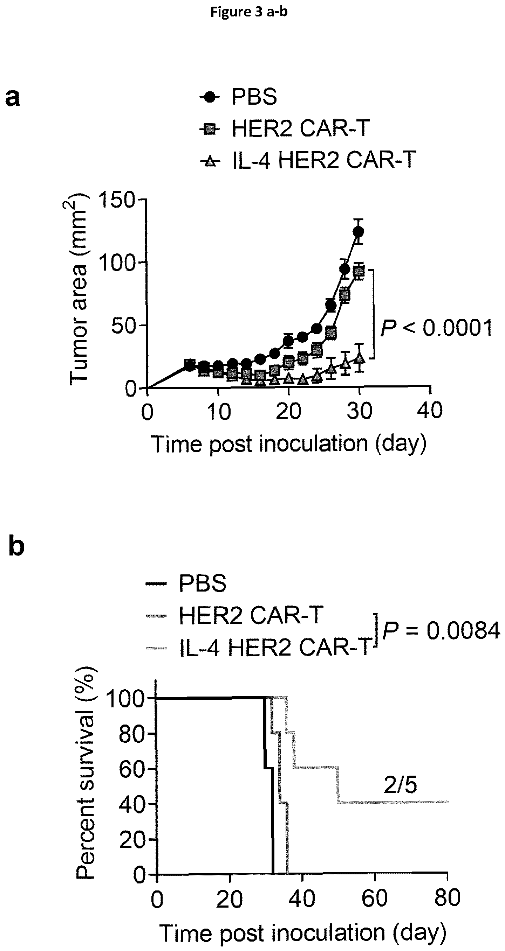

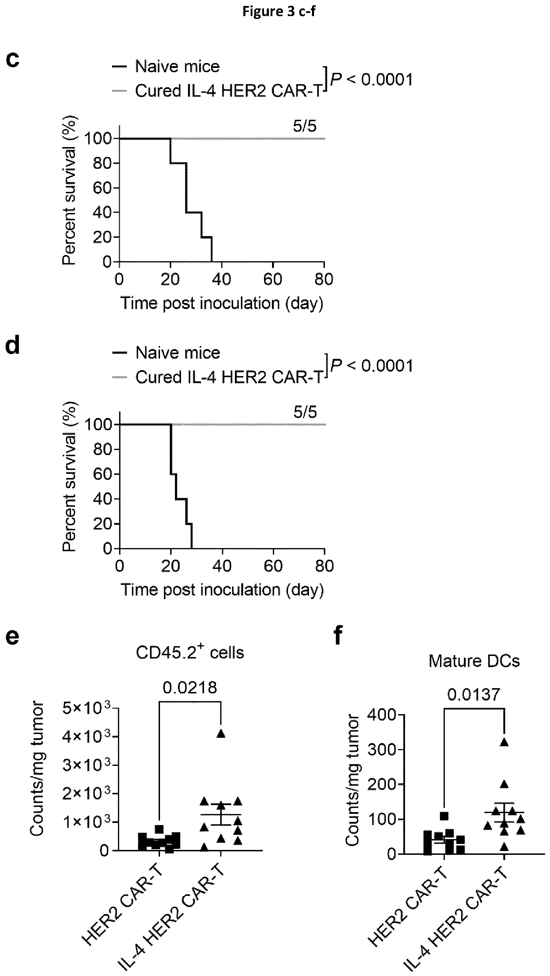

**Figure 3****. IL-4 HER2 CAR T therapy eradicates established mouse MC38-HER2 colon adenocarcinoma, induces durable antitumor immunity and broad immune activation.** a,b, C57BL/6 mice were inoculated (s.c.) with MC38-HER2 colon cancer cells (3 × 10⁵), sublethally lymphodepleted by irradiation on day 5, and received i.v. adoptive transfer of IL-4 HER2 CAR-T cells (3 × 10⁶), or HER2 CAR-T cells (3 × 10⁶) on day 6 (n = 5 per group). a,b, Shown are average tumor growth curves (a) and mouse survival curves (b) of MC38-HER2 model. Shown are numbers of long-term-surviving mice among the total number of mice in the group (b). c,d, Survivors post the treatment of IL-4 HER2 CAR-T cells were rechallenged s.c. with MC38-HER2 (1 × 10⁶) on day 90 post primary tumor inoculation. The Survivors post rechallenged s.c. with MC38-HER2 were further rechallenged s.c. with MC38 (0.5 × 10⁶) on day 130 post primary tumor inoculation. Naive WT mice (n = 5 per group) were inoculated with the same number of tumor cells as controls. c,d, Shown are survival curves and numbers of long-term survivors rejecting the second tumor challenge in MC38-HER2 (c) and MC38 (d) tumor models. Shown are survival curves and numbers of long-term-surviving mice against the re-challenges (c, d). e,f, C57BL/6 mice were inoculated with MC38-HER2 tumor cells (1 × 10⁶, s.c.), sublethally lymphodepleted by irradiation on day 5, and received i.v. adoptive transfer of HER2 CAR-T cells (3 × 10⁶) or IL-4 HER2 CAR-T cells (3 × 10⁶) on day 6. On day 14, mice were killed for phenotype analyses of indicated immune cells in tumors by flow cytometry. Shown are counts of CD45.2⁺ cells (e) and mature dendritic cells (DCs) (f) in the MC38-HER2 tumors from each treatment group. All data represent the mean ± s.e.m. and are analyzed by unpaired Student's t-test (e,f), ANOVA with Tukey's multiple-comparisons test (a), or log-rank test (b-d).

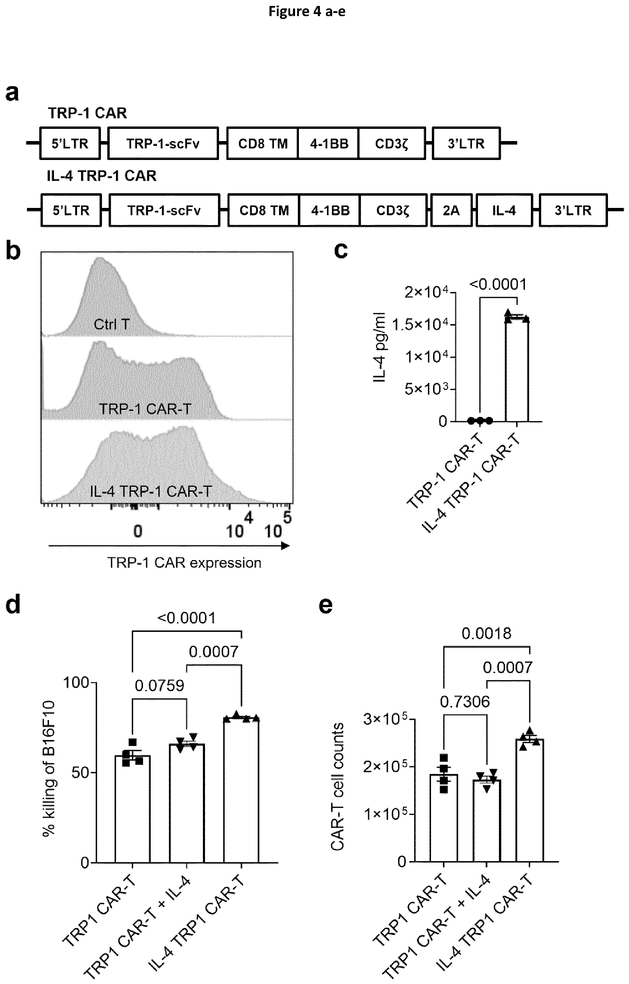

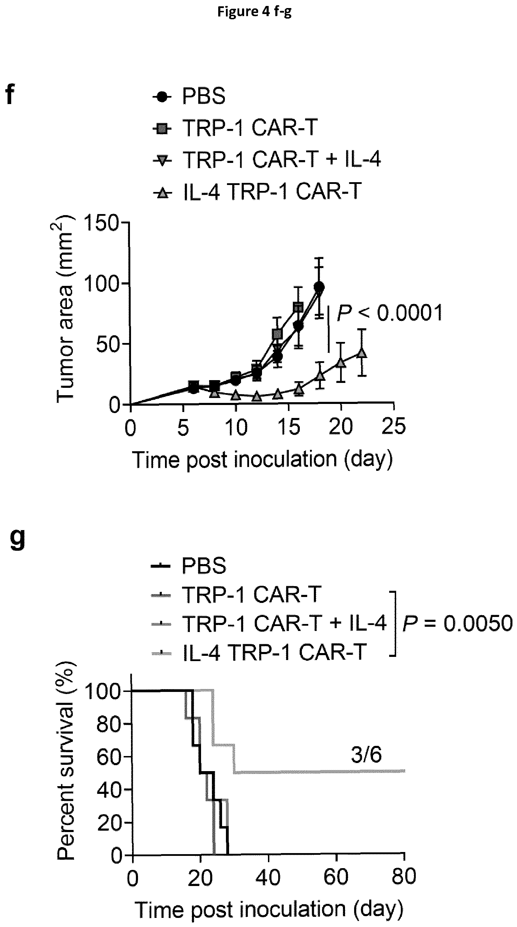

**Figure 4****. Complete regression of pre-established mouse B16F10 melanoma model by treatment with IL-4 TRP-1 CAR-T cells,** a, Schematic depicting constructs of TRP-1-directed second-generation 4-1BB-based CAR (TRP-1 CAR) and murine IL-4-secreting TRP-1 CAR (IL-4 TRP-1 CAR), b, The expression levels of TRP-1 CAR were examined by staining the c-Myc tag. Ctrl T, untransduced T cells as control. c, IL-4 TRP-1 CAR-T or TRP-1 CAR-T cells were cultured for 2 days. The culture supernatants were examined for the concentration of IL-4 by ELISA. d,e, IL-4 TRP-1 CAR-T or TRP-1 CAR-T cells in the absence or presence of IL-4 (20 ng/ml) were cocultured with B16F10 cells at the E:T ratio of 0.5:1 for 48 h. The percentage of tumor cell lysis (d) and viable counts of CAR-T cells (e) was analyzed by flow cytometry. f,g, C57BL/6 mice were inoculated (s.c.) with B16F10 melanoma cells (3 × 10⁵), lymphodepleted, and received i.v. adoptive transfer of IL-4 TRP-1 CAR-T cells (3 × 10⁶), or TRP-1 CAR-T cells (3 × 10⁶) in the presence or absence of i.v. administered IL-4 (1 µg) on day 6 (n = 6 per group). Shown are average tumor growth curves (f) and mouse survival curves (g) of B16F10 model. Shown are numbers of long-term-surviving mice among the total number of mice in the group (g). All data represent the mean ± s.e.m. and are analyzed by unpaired Student's t-test (c), ANOVA with Tukey's multiple-comparisons test (d, e, f), or log-rank test (g).

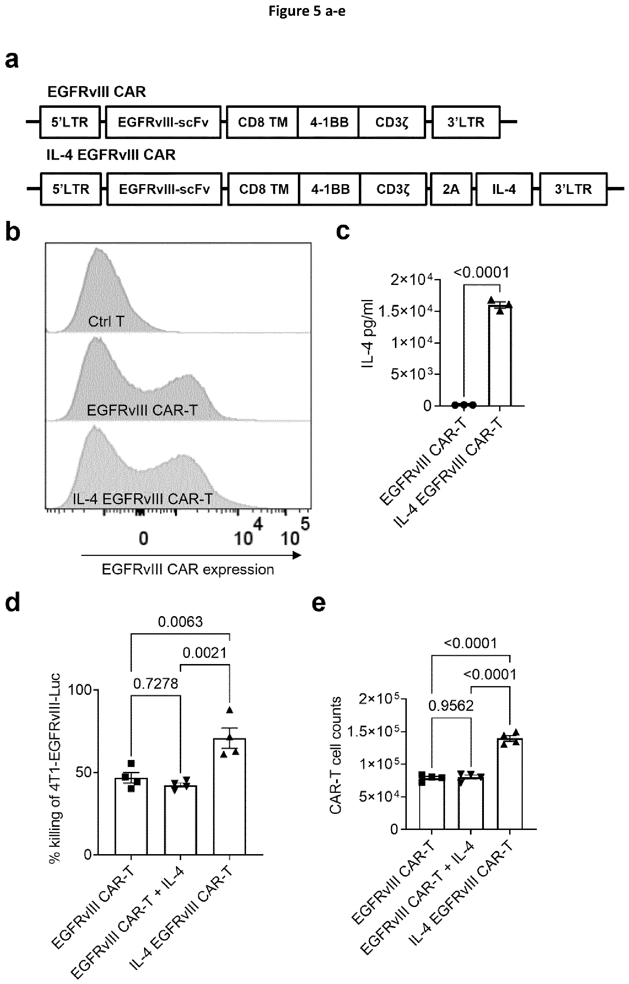

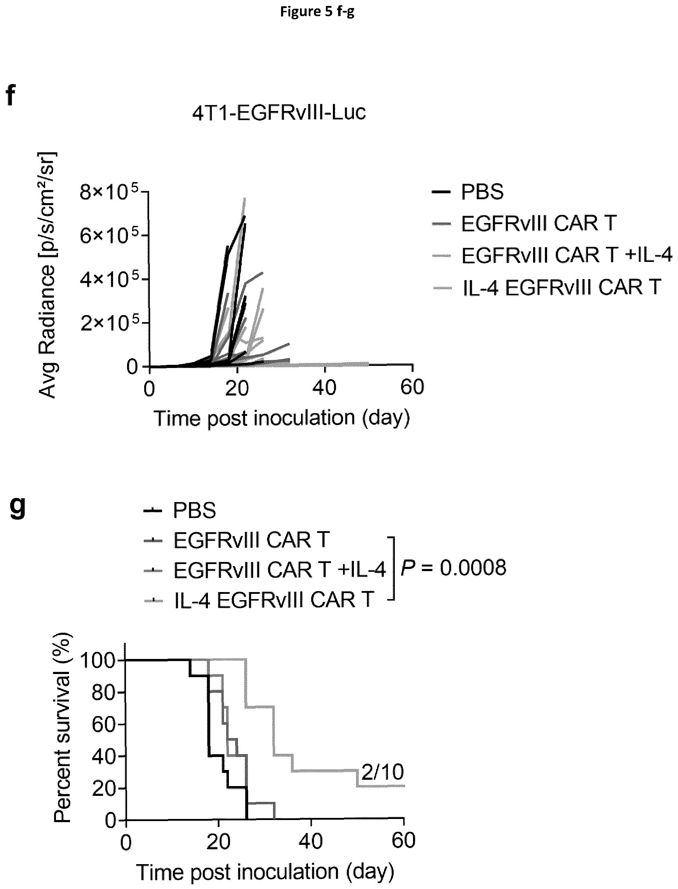

**Figure 5****. IL-4 EGFRvIII CAR T therapy prolong survival in pre-established mouse 4T1-Luc-EGFRvIII metastatic mammary carcinoma model.** a, Schematic depicting constructs of EGFRvIII-directed second-generation 4-1BB-based CAR (EGFRvIII CAR) and murine IL-4-secreting EGFRvIII CAR (IL-4 EGFRvIII CAR), b, The expression levels of EGFRvIII CAR were examined by staining the c-Myc tag. Ctrl T, untransduced T cells as control. c, IL-4 EGFRvIII CAR-T or EGFRvIII CAR-T cells were cultured for 2 days. The culture supernatants were examined for the concentration of IL-4 by ELISA. d,e, IL-4 EGFRvIII CAR-T or EGFRvIII CAR-T cells in the absence or presence of IL-4 (20 ng/ml) were cocultured with 4T1-EGFRvIII-Luc cells at the E:T ratio of 0.5:1 for 48 h. The percentage of tumor cell lysis (d) and viable counts of CAR-T cells (e) was ana-lyzed by flow cytometry. f,g, BALB/c mice were inoculated (i.v.) with 4T1-EGFRvIII-Luc breast cancer cells (5 × 10⁴), lymphodepleted, and received i.v. adoptive transfer of IL-4 EGFRvIII CAR-T cells (3 × 10⁶), or EGFRvIII CAR-T cells (3 × 10⁶) in the presence or absence of i.v. administered IL-4 (1 µg) on day 6 (n = 10 per group). f, Individual radiance (p/s/cm2/sr) of different treatment groups of mice. g, Survival curves of 4T1-EGFRvIII-Luc tumor model. Shown are numbers of long-term-surviving mice among the total number of mice in the group (g). All data represent the mean ± s.e.m. and are analyzed by unpaired Student's t-test (c), ANOVA with Tukey's multiple-comparisons test (d, e), or log-rank test (g).

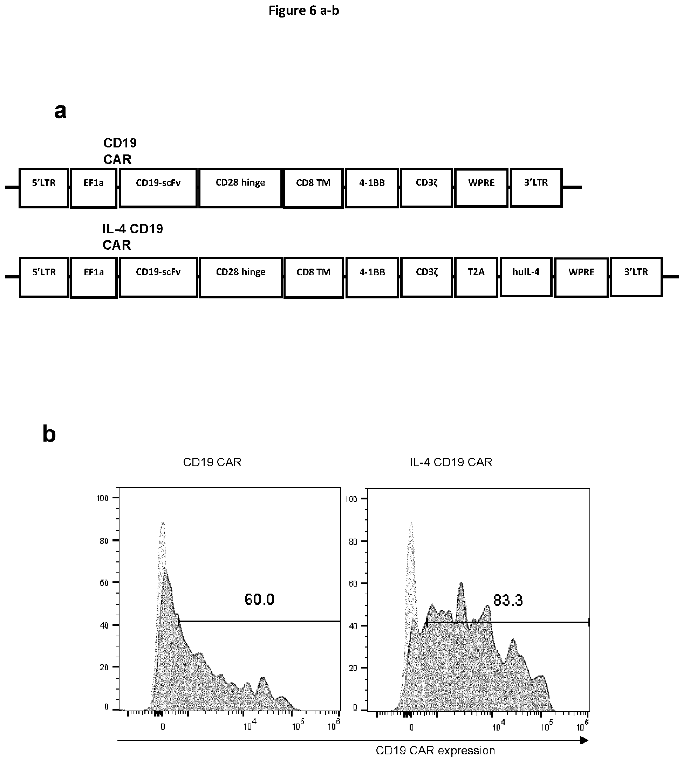

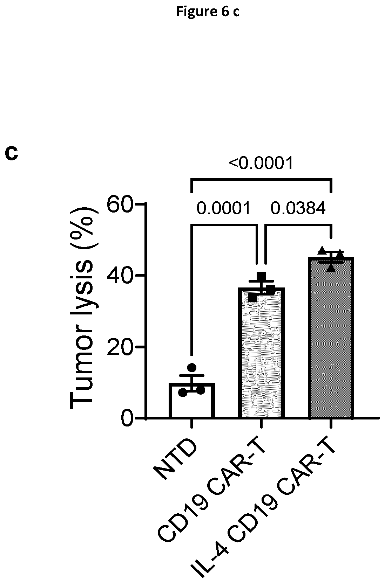

**Figure 6****. In vitro characterization of IL-4 CD19 human CAR-T.** a, Schematic representations of CD19-directed second generation CAR (CD19 CAR), and CD19-directed second generation CAR modified to express human IL-4. b, The expression levels of CAR were analyzed by flow cytometry. d, NTD (nontransduced T cells), CD19 CAR-T cells, or CD19 IL-4 CAR-T cells co-culture with Nalm6-GFP cells at the E:T ratio of 1:5 for 48 hours, tumor cell killing percentage were analyzed by flow cytometry. Data represent the mean ± SEM.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of "include/including", that is to say permitting the presence of one or more features or components. This term also encompasses the more restricted term "consist/consisting of".

As used in the specification and claims, the singular form "a", "an" and "the" include plural references unless the context clearly dictates otherwise.

The term "amino acid" includes all of the naturally occurring amino acids as well as modified amino acids.

As used herein, "at least one" means "one or more", "two or more", "three or more", etc. For example, at least one costinulatroy domain means one costinulatroy domain, two costinulatroy domains, three costinulatroy domains or so.

The term "about" particularly in reference to a given quantity, is meant to encompass deviations of plus or minus ten (10) percent (%).

"Homology" refers to the percent identity between two polynucleotide or two polypeptide moieties. Two nucleic acid, or two polypeptide sequences are "substantially homologous" to each other when the sequences exhibit at least about 50% sequence identity, preferably at least about 75% sequence identity, more preferably at least about 80% or at least about 85% sequence identity, more preferably at least about 90% sequence identity, and most preferably at least about 95%-98% sequence identity over a defined length of the molecules. As used herein, substantially homologous also refers to sequences showing complete identity to the specified sequence. Alternatively, homology can be determined by readily available computer programs or by hybridization of polynucleotides under conditions which form stable duplexes between homologous regions, followed by digestion with single stranded specific nuclease(s), and size determination of the digested fragments. DNA sequences that are substantially homologous can be identified in a Southern hybridization experiment under, for example, stringent conditions, as defined for that particular system. Defining appropriate hybridization conditions is within the skill of the art.

In general, "identity" refers to an exact nucleotide to nucleotide or amino acid to amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100. In some aspects, a nucleotide or amino acid sequence of the invention, or a portion thereof, is at least 80%, namely, 80%, 81%, 82%, 83%, 84%, 85%, 86%, 87%, 88%, 89%, 90%, 91%, 92%, 93%, 94%, 95%, 96%, 97%, 98%, or 99% identical to a corresponding nucleotide or amino acid sequence (SEQ ID NO identifier).

As used herein, the terms "peptide", "protein", "polypeptide", "polypeptide chain", "polypeptidic" and "peptidic" are used interchangeably to designate a series of amino acid residues connected to the other by peptide bonds between the alpha-amino and carboxy groups of adjacent residues.

The term "variant", when it refers to a polypeptide or nucleic acid of the invention means one or more biologically active derivatives of a polypeptide or nucleic acid. In general, the term "variant" refers to molecules having a native sequence with one or more additions, substitutions (generally conservative in nature) and/or deletions, relative to the native molecule, so long as the modifications do not destroy its biological activity and which are "substantially homologous" to the reference molecule (Gorby et al., Sci. Signal. 13, eabc0653, 2020; Saxton et al., Science 371, eabc8433, 2021). In general, the sequences of such variants will have a high degree of sequence homology or identity to the reference sequence, e.g., sequence homology or identity of more than 25%, generally more than 50% to 70%, even more particularly 80%, or 85% or more, such as at least 90%, or 95% or more, when the two sequences are aligned. Spencer, Juliet V et al. reported that splicing forms of IL-10 retain biological activities or properties, despite having only 27% sequence identity to hIL-10 (Spencer, Juliet V et al. "Stimulation of B lymphocytes by cmvIL-10 but not LAcmvIL-10." Virology vol. 374,1 (2008): 164-9. doi:10.1016/j.virol.2007.11.031, the contents of which are hereby incorporated by reference in their entirety).

As used herein, a "fragment" of a polypeptide or nucleic acid of the invention refers to a sequence containing less nucleotides in length than the respective nucleic acid sequence or less amino acids in length that the polypeptide sequence. Preferably, this sequence or fragment contains less than 90%, preferably less than 60%, in particular less than 30% nucleotides or amino acids in length than the respective nucleic acid sequence or respective polypeptide sequence. More preferably, the "fragment" of a polypeptide or nucleic acid of the invention is a "functional fragment", that means that it shares the same biological functions or properties than the respective nucleic acid sequence or respective polypeptide sequence.

The Inventors of the present invention have shown that, surprisingly, CAR-T cells engineered to express a type 2 cytokine, such as e.g. IL-4, exhibited enhanced proliferative capacity, effector function, and prevented the functional impairment in e.g. solid tumors when compared to CAR-T cells not expressing an IL-4.

The present invention provides a chimeric antigen receptor (CAR) polypeptide comprising at least one antigen binding domain, a transmembrane domain, at least one costimulatory domain, at least one activating domain, and at least one type 2 cytokine, a variant or a functional fragment thereof. Preferably, the at least at least one type 2 cytokine is an interleukin 4 (IL-4) molecule, a variant or a functional fragment thereof

Bispecific and multispecific CARs are contemplated within the scope of the invention, with specificity to more than one target of interest.

Optionally, short linkers may form linkages between any or some of the domains, namely between any or some of the antigen binding domain, transmembrane domain, costimulatory domain, activating domain, intracellular domain, and IL-4 molecule of the CAR.

The antigen binding domain usually consists of, or is comprised within, an extracellular domain that binds to a cell surface marker. Preferably, the antigen binding domain is selected from the non-limiting group comprising an antigen-binding polypeptide, a receptor, and a natural ligand for a target cell antigen or receptor.

Preferably, the antigen-binding polypeptide is an antibody or antibody fragment, selected from the group comprising murine antibodies, rabbit antibodies, human antibodies, humanized antibodies, single chain variable fragments (scFv), camelid antibody variable domains and humanized versions, shark antibody variable domains and humanized versions, single domain antibody variable domains, nanobodies (VHHs), and camelized antibody variable domains.

In one aspect, the antigen binding domain is an extracellular antigen recognition or binding domain of a single-chain Fragment variant (scFv) derived from an antibody recognizing an antigen, a ligand or a receptor.

In one aspect, the antigen recognized by the antigen binding domain is usually selected from the group comprising a cancer cell associated antigen, an infection-associated antigen and an autoantigen.

Preferably, the antigen recognized by the extracellular antigen binding domain is selected from the group comprising HER2, TRP-1, EGFRvIII, CD19, CD20, CD38, CD30, ERBB2, fibroblast activation protein (FAP), CA125, MUC-1, PSMA, PSA, CD44 surface adhesion molecule, mesothelin, carcinoembryonic antigen (CEA), CEACAM5, CEACAM6, epidermal growth factor receptor (EGFR), vascular endothelial growth factor receptor-2 (VEGFR2), high molecular weight-melanoma associated antigen (HMW-MAA), MAGE-A1, IL-13R-a2, GD2, carbonic anhydrase EX, alpha-fetoprotein, A3, antigen specific for A33 antibody, Ba 733, BrE3-antigen, CD1, CDIa, CD3, CD5, CD15, CD16, CD19, CD20, CD21, CD22, CD23, CD25, CD30, CD33, CD38, CD45, CD74, CD79a, CD80, CD138, colon-specific antigen-p (CSAp), CSAp, EGP-I, EGP-2, Ep-CAM, FIt-I, Flt-3, folate receptor, HLA-DR, human chorionic gonadotropin (HCG) and its subunits, hypoxia inducible factor (HIF-I), Ia, IL-2, IL-6, IL-8, insulin growth factor-1 (IGF-I), KC4-antigen, KS-1-antigen, KS1-4, Le-Y, macrophage inhibition factor (MIF), MAGE, MUC1, MUC2, MUC3, MUC4, NCA66, NCA95, NCA90, tyrosinase, PRAME, EBNA, KLK3, HPV E7, LMP2, NY-ESO-1, PAP, reverse transcriptase, nucleophosmin, PRTN3/ELANE, CT83/KKLC1, MUC16, DNTT, antigen specific for PAM-4 antibody, placental growth factor, p53, prostatic acid phosphatase, RS5, S1OO, TAC, TAG-72, tenascin, TRAIL receptors, Tn antigen, Thomson-Friedenreich antigens, tumor necrosis antigens, VEGF, ED-B fibronectin, 17-1A-antigen, NeuGcGM3, N-glycolyl GM3 ganglioside, Neu5Gc, GM3-Ganglioside, GD3, GM2, carbohydrate antigens, ganglioside antigens, Lewis Y, Lewis B, MOG, MBP, aB-crystallin, PLP, GlialCAM, β-synuclein, HLA-A2, TNP, CD123, Kappa chain of immunoglobulin, or any combination thereof.

Non-limiting examples of scFv are selected from the group comprising HER2-scFv (SEQ ID NO:3), TRP-1-scFv (SEQ ID NO:4), and EGFRvIII-scFv (SEQ ID NO:5), a fragment or variant of any one of these sequences.

In some instances, the antigen binding domain comprises a hinge portion (also called linker or spacer). A variety of hinges or linkers can be employed in accordance with the invention as described herein and known in the art.

One of ordinary skills in the field will appreciate that any suitable transmembrane domain can be used in this invention. In one aspect, the transmembrane domain can be selected from the group comprising CD28, CD28T, OX-40, 4-1BB/CD137, CD2, CD7, CD27, CD30, CD40, programmed death-1 (PD-1), inducible T cell costimulator (ICOS), CDl-la/CD18, CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD( (e.g. CD8alpha, CD8beta), IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD 162), LTBR, LAT, GADS, SLP-76, PAG/Cbp, CD 19a, a ligand that specifically binds with CD83, or any combination thereof. Preferably, the transmembrane domain is selected from the group comprising the CD8 transmembrane, a variant or fragment thereof.

Where a linker region is interposed between the antigen binding domain and the transmembrane domain, it will be selected from the group comprising i) an immunoglobulin hinge region or a linker region derived from CD8, CD8α, or CD28, or any variant thereof, and ii) a (GnS)m linker, wherein G is glycine, S is serine,n is an integer between 2-12, preferably between 3-10, more preferably between 3-5, even more preferably n=3 and m is an integer between 2-12, preferably between 3-10, more preferably between 3-5, even more preferably m=3.

In one aspect, the CD8 transmembrane domain and hinge region is as set forth in SEQ ID NO: 8, a variant or a fragment thereof.

The costimulatory domain is a signalling region (or other suitable portion) comprising, or consisting of, a molecule selected from CD28, OX-40, 4-1BB/CD137, CD2, CD7, CD27, CD30, CD40, programmed death-1 (PD-1), inducible T cell costimulator (ICOS), lymphocyte function-associated antigen-1 (LFA-1 (CDl la/CD18), CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class I molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, or any combination thereof.

Preferably, the costimulatory domain is selected from the group comprising the 4-1BB costimulatory domain, a variant or fragment thereof. Most preferably, the 4-1BB costimulatory domain is as set forth in SEQ ID NO: 9, a variant or a fragment thereof.

A suitable source of activating domain can be derived from (or correspond to) some or all of CD3 .

In a preferred aspect, the activating domain is a CD3 domain, more preferably a CD3 zeta (CD3ζ ) domain.

Most preferably, the CD3ζ domain is as set forth in SEQ ID NO: 8, a variant or a fragment thereof.

The CAR of the invention also comprises a type 2 cytokine. Type 2 cytokines include interleukin-4 (IL-4), IL-5, IL-6, IL-9, IL-13, IL-25 and IL-33.

In one aspect, the type 2 cytokine is an interleukin 4 (IL-4) molecule, a variant or a functional fragment thereof.

Preferably, the IL-4 is as set forth in SEQ ID NO: 1 or SEQ ID NO: 2, a variant or a fragment of any one of these sequences.

In one aspect, the linking between the activating domain and IL-4 is, e.g., via a sequence encoding a self-cleaving peptide. Preferably, the self-cleaving domain or peptide comprises the sequence DxExNPGP (wherein x is any amino acid).

Non-limiting examples of a self-cleaving domain or peptide comprising the sequence DxExNPGP is selected from the group comprising:

| | | |
|---|---|---|
| T2A | EGRGSLLTCGDVEENPGP | SEQ ID NO. 10 |
| P2A | ATNFSLLKQAGDVEENPGP | SEQ ID NO. 11 |
| E2A | QCTNYALLKLAGDVESNPGP | SEQ ID NO. 12 |
| F2A | VKQTLNFDLLKLAGDVESNPGP | SEQ ID NO. 13, |

or a combination thereof.

For example, once the self-cleaving peptide is cleaved, IL-4, a fragment or a variant thereof, is secreted or membrane bond by, or on, the immune cell, preferably in the tumor microenvironment.

As will be appreciated, adding the optional linker "GSG" (Gly-Ser-Gly) on the N-terminal of a 2A peptide of the invention may help with efficiency.

The invention further contemplates an isolated polynucleotide, or nucleic acid, encoding the chimeric antigen receptor (CAR) of the invention, as well as a fragment or a variant thereof, including a domain or region disclosed herein.

The terms "nucleic acid", "polynucleotide," and "oligonucleotide" are used interchangeably and refer to any kind of deoxyribonucleotide (e.g. DNA, cDNA, ...) or ribonucleotide (e.g. RNA, mRNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity, i.e., an analogue of A will base-pair with T.

Further contemplated is a vector comprising an isolated polynucleotide of the invention. The term "vector", as used herein, refers to any vector known in the art that can be suitable for the present invention. In some aspects, the vector refers to a viral vector or to a nucleic acid (DNA or RNA) molecule such as a plasmid or other vehicle, which contains one or more heterologous nucleic acid sequence(s) of the invention and, preferably, is designed for transfer between different host cells and/or for amplification purposes. The terms "expression vector", "gene delivery vector" and "gene therapy vector" refer to any vector that is effective to incorporate and express one or more nucleic acid(s) of the invention, in a cell, preferably under the regulation of a promoter. A cloning or expression vector may comprise additional elements, for example, regulatory and/or post-transcriptional regulatory elements in addition to a promoter.

In one aspect, the vector is selected from the non-limiting group comprising a retroviral vector, a DNA vector, a plasmid, an RNA vector, an adenoviral vector, an adenovirus associated vector, a lentiviral vector, an RNA (e.g. mRNA) targeted lipid nanoparticles (LNPs), a liposome or any combination thereof.

The present invention further provides a host cell comprising and/or expressing at least one isolated nucleic acid of the invention, at least one expression vector of the invention and/or at least one CAR of the invention.

In one aspect, the cell is a mammalian cell, whether an autologous or an allogeneic cell. Preferably, the cell is selected from the group comprising a cytotoxic cell, an immune cell, a stem cell, a progenitor cell, a cell line and a cell derived from a stem cell or a progenitor cell.

Where the cell is an immune cell, said immune cell will be selected from the non-limiting group comprising T cell, tumor infiltrating lymphocyte (TIL), NK cell, regulatory T cell (Treg cell), macrophage, TCR-expressing cell, eosinophil, basophil, neutrophil, myeloid cells, B cell, plasma cell, regulatory B cell (Breg), innate lymphoid cell 1 (ICL1), ILC2, ICL3, dendritic cell, or NK-T cell.

There are a variety of available techniques know in the art for isolation and enrichment of T cells, such as e.g. PBMC obtained from peripheral blood samples. Preferably, the immune cell is selected from the group comprising a T cell, tumor infiltrating lymphocyte (TIL), NK cell, TCR-expressing cell, dendritic cell, or NK-T cell

Where the immune cell is a T-cell or NK cell, it will be either an autologous or an allogeneic T cell or NK cell. In some aspects, the immune cell is a T-cell line or NK cell line. Any cell line derived from any primary cell can be used in the present invention. NK cells can, for example, be engineered from various NK sources such as iPSC-derived NK cells, NK cells isolated from blood (PB-NK), NK isolated from the umbilical cord blood or NK cell lines comprising NK92, YTS or KHYG1 cell lines.

Where the cell is derived from a stem cell, it will be either a natural or an induced stem cell (e.g., an iPSC-derived cell).

According to an aspect of the invention, the immune cell expresses an interleukin-4, a fragment or a variant thereof. Preferably, the immune cell comprises one or more recombinant constructs, wherein at least one recombinant construct encodes an interleukin-4 (IL-4), a fragment or a variant thereof.

The at least one recombinant construct encodes a chimeric antigen receptor (CAR), a T cell receptor (TCR) or any other synthetic tumor targeting motif described herein.

According to one aspect, the construct encoding an IL-4, a fragment or a variant thereof encodes i) a sequence comprising, or consisting of, SEQ ID NO. 1, a variant or a functional fragment thereof or ii) a sequence comprising, or consisting of, SEQ ID NO. 2, a variant or a functional fragment thereof.

Preferably, the recombinant construct encoding an IL-4, a variant or a functional fragment thereof is linked to the second recombinant construct encoding a CAR, a TCR, or any other synthetic tumor targeting motif. In one aspect, the linking is via a sequence encoding a self-cleaving peptide (e.g. peptide 2A).

Alternatively, the host cell (e.g. immune cell) comprises and/or expresses at least one expression vector of the invention and at least one CAR, a TCR, or any other synthetic tumor targeting motif of the invention. In this case, the expression vector expresses a first recombinant construct encoding an IL-4, a variant or a functional fragment thereof whereas the second recombinant construct encodes a CAR, a TCR, or any other synthetic tumor targeting motif but does not encode an IL-4, a variant or a functional fragment thereof.

The present invention provides compositions comprising i) a chimeric antigen receptor polypeptide of the invention, ii) an isolated polynucleotide, or nucleic acid, of the invention, iii) a vector of the invention, or iv) a host cell, such as e.g. an immune cell expressing a chimeric antigen receptor according to the invention.

The present invention also provides pharmaceutical compositions comprising a therapeutically effective amount of i) a chimeric antigen receptor polypeptide of the invention, ii) an isolated polynucleotide, or nucleic acid, of the invention, iii) a vector of the invention, or iv) a host cell, such as e.g. an immune cell expressing a chimeric antigen receptor according to the invention, and pharmaceutically acceptable carrier, diluent and/or excipient.

The present invention further provides the use of pharmaceutical compositions comprising a therapeutically effective amount of i) a chimeric antigen receptor polypeptide of the invention, ii) an isolated polynucleotide, or nucleic acid, of the invention, iii) a vector of the invention, or iv) a host cell, such as e.g. an immune cell expressing a chimeric antigen receptor according to the invention, and pharmaceutically acceptable carrier, diluent and/or excipient in the manufacture of a medicament for treating a cancer, an infectious disease, an inflammatory or inflammation-induced disease, a chronic disease or an autoimmune disease.

The term "therapeutically effective amount" as used herein means an amount of an immune cell, CAR polypeptide, nucleic acid, plasmid or vector, high enough to significantly positively modify the symptoms and/or condition to be treated, but low enough to avoid serious side effects (at a reasonable risk/benefit ratio), within the scope of sound medical judgment.

The therapeutically effective amount of a host cell, e.g. an immune cell, Car polypeptide, nucleic acid, plasmid or vector as described herein is selected in accordance with a variety of factors including type, species, age, weight, sex and medical condition of the patient or subject; the severity of the condition or disease (e.g. cancer, infection or autoimmune disease ) to be treated; the route of administration; the renal and hepatic function of the patient or subject. A physician of ordinary skill in the art can readily determine and prescribe the effective amount of the immune cell, nucleic acid, plasmid of vector required to prevent, counter or arrest the progress of the disease, such as e.g. a cancer, an infectious disease, an inflammatory or inflammation-induced disease, a chronic disease or an autoimmune disease.

"Pharmaceutically acceptable carrier or diluent" means a carrier or diluent that is useful in preparing pharmaceutical compositions that is generally safe, non-toxic, and desirable, and includes carriers or diluents that are acceptable for human pharmaceutical use.

Such pharmaceutical compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

Any of the compositions provided herein can be provided in any appropriate pharmaceutical composition and be administered by any suitable route of administration. Suitable routes of administration include, but are not limited to, inhalation, intra-arterial, intradermal, intramuscular, intraperitoneal, intravenous, nasal, parenteral, pulmonary, and subcutaneous routes. Pharmaceutical compositions of the present invention are preferably formulated for intravenous administration.

The pharmaceutical compositions (solutions, suspensions or the like), may include one or more of the following: sterile diluents such as water for injection, saline solution, preferably physiological saline, Ringer's solution, isotonic sodium chloride, fixed oils such as synthetic mono- or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. An injectable pharmaceutical composition is preferably sterile.

The pharmaceutical compositions of the invention can further comprise at least one additional therapeutic agent or therapy. A variety of other additional therapeutic agents may be used in conjunction with the compositions described herein.

In one aspect, said at least one additional therapeutic agent or therapy is an anticancer agent or anticancer therapy, useful to treat a cancer, preferably a solid cancer. Preferably, the one or more anti-cancer therapy will be selected from the group comprising radiotherapy, chemotherapy, immune checkpoint inhibitor, immunotherapy and hormone therapy, or a combination of one of more thereof.

Additional therapeutic agents suitable for use in combination with the invention include, but are not limited to, ibrutinib (Imbruvica^{®}), ofatumumab (Arzerra^{®}), rituximab (Rituxan^{®}), bevacizumab (Avastin^{®}), trastuzumab (Herceptin^{®}), trastuzumab emtansine (KADCYLA^{®}), imatinib (Gleevec^{®}), cetuximab (Erbitux^{®}), panitumumab (Vectibix^{®}), catumaxomab, ibritumomab, ofatumumab, tositumomab, brentuximab, alemtuzumab, gemtuzumab, erlotinib, gefitinib, vandetanib, afatinib, lapatinib, neratinib, axitinib, masitinib, pazopanib, sunitinib, sorafenib, toceranib, lestaurtinib, axitinib, cediranib, lenvatinib, nintedanib, pazopanib, regorafenib, semaxanib, sorafenib, sunitinib, tivozanib, toceranib, vandetanib, entrectinib, cabozantinib, imatinib, dasatinib, nilotinib, ponatinib, radotinib, bosutinib, lestaurtinib, ruxolitinib, pacritinib, cobimetinib, selumetinib, trametinib, binimetinib, alectinib, ceritinib, crizotinib, aflibercept, adipotide, denileukin diftitox, mTOR inhibitors such as Everolimus and Temsirolimus, hedgehog inhibitors such as sonidegib and vismodegib, CDK inhibitors such as CDK inhibitor (palbociclib).

In additional aspects, the additional therapeutic agent can be an anti-inflammatory agent. Anti-inflammatory agents or drugs include, but are not limited to, steroids and glucocorticoids (including betamethasone, budesonide, dexamethasone, hydrocortisone acetate, hydrocortisone, hydrocortisone, methylprednisolone, prednisolone, prednisone, triamcinolone), nonsteroidal anti-inflammatory drugs (NSAIDS) including aspirin, ibuprofen, naproxen, methotrexate, sulfasalazine, leflunomide, anti-TNF medications, cyclophosphamide and mycophenolate. Exemplary NSAIDs include ibuprofen, naproxen, naproxen sodium, Cox-2 inhibitors, and sialylates. Exemplary analgesics include acetaminophen, oxycodone, tramadol of proporxyphene hydrochloride. Exemplary glucocorticoids include cortisone, dexamethasone, hydrocortisone, methylprednisolone, prednisolone, or prednisone. Exemplary biological response modifiers include molecules directed against cell surface markers (e.g., CD4, CDS, etc.), cytokine inhibitors, such as the TNF antagonists, (e.g., etanercept (ENBREL^{®}), adalimumab (HUMIRA^{®}) and infliximab (REMICADE^{®}), chemokine inhibitors and adhesion molecule inhibitors. The biological response modifiers include monoclonal antibodies as well as recombinant forms of molecules. Exemplary DMARDs include azathioprine, cyclophosphamide, cyclosporine, methotrexate, penicillamine, leflunomide, sulfasalazine, hydroxychloroquine, Gold (oral (auranofin) and intramuscular) and minocycline.

The present invention further contemplates methods of treating and/or preventing a cancer, an infectious disease, an inflammatory or inflammation-induced disease, a chronic disease or an autoimmune disease comprising administering i) an isolated polynucleotide of the invention, ii) a vector of the invention, iii) a host cell, e.g. an immune cell of the invention, or iv) a pharmaceutical composition of the invention, to a subject in need thereof.

The term "treatment" or "treating" means any administration of a composition, pharmaceutical composition, therapeutic agent, compound, *etc...* of the disclosure to a subject for the purpose of:
(i) inhibiting the disease, that is, arresting the development of clinical symptoms; and/or
(ii) relieving the disease, that is, causing the regression of clinical symptoms.

As used herein, the term "prevention" or "preventing" means any administration of a composition, pharmaceutical composition, therapeutic agent, compound, *etc...* of the disclosure to a subject for the purpose of preventing the disease, that is, causing the clinical symptoms of the disease not to develop.

In the context of the present invention, the disease is a cancer, an infectious disease, an inflammatory or inflammation-induced disease, a chronic disease or an autoimmune disease .

As used herein the terms "subject"/"subject in need thereof", or "patient"/"patient in need thereof " are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered. Preferably, the subject is a human, most preferably a human that suffer or might be at risk of suffering from a cancer, an infectious disease, an inflammatory or inflammation-induced disease, a chronic disease or an autoimmune disease.

In one aspect, the method of treatment and/or prevention of a cancer, an infection or an autoimmune disease in a patient or subject comprises (i) removing and isolating immune cells, preferably immune cells, more preferably native T cells, from said patient or subject, (ii) genetically engineering said T cells with one recombinant construct (e.g. vector, plasmid or polynucleotide) encoding a recombinant construct encoding a chimeric antigen receptor (CAR) or the invention, (iii) expanding *ex vivo* into a larger population of engineered immune cells, e.g. T cells, and (iv) reintroducing said engineered immune cells, e.g. T cells, into the patient or subject. After the immune cells, e.g. T cells are reintroduced into the patient or subject, they exert their function, which varies depending on the immune cell type used. Examples of functions are triggering cell death on the target cells (T and NK), phagocytosis of the target cell (macrophages), or induce an immunosuppressive environment around their target (Tregs).

Alternatively, the method of treatment and/or prevention of a cancer, an infectious disease, an inflammatory or inflammation-induced disease, a chronic disease or an autoimmune disease in a patient or subject comprises (i) providing genetically engineered immune cells such as e.g. T cells cells with one recombinant construct (e.g. vector, plasmid or polynucleotide of the invention) encoding a chimeric antigen receptor (CAR) or the invention, (ii) expanding ex *vivo* into a larger population of engineered immune cells, e.g. T cells, and (iii) reintroducing said engineered immune cells, e.g. T cells, into the patient or subject.

Where the disease to be treated is an inflammatory or inflammation-induced disease, it is selected from the non-limiting group comprising fibrosis (lung or cardiac fibrosis), chronic obstructive pulmonary disease, cardiovascular diseases, diabetes, asthma, fatty liver disease, gout, and scleroderma.

Where the disease to be treated is a chronic disease, it is selected from the non-limiting group comprising fibrosis, Alzheimer disease, lupus erythematosus, and chronic kidney disease.

Where the disease to be treated is an infectious disease, it is selected from the non-limiting group comprising HIV, Hepatitis C and Human Cytomegalovirus.

Where the disease to be treated is cancer, it is selected from a solid cancer or a liquid cancer.

Where the cancer to be treated is solid, it is selected from the non-limiting group comprising lung cancer, breast cancer, ovarian cancer, cervical cancer, uterus cancer, head and neck cancer, glioblastoma, hepatocellular carcinoma, colon cancer, rectal cancer, colorectal carcinoma, kidney cancer, prostate cancer, gastric cancer, bronchus cancer, pancreatic cancer, urinary bladder cancer, hepatic cancer and brain cancer and skin cancer, in particular melanoma, or a combination of one or more thereof.

Where the cancer to be treated is liquid it refers to cancer cells that are present in body fluids, such as blood, lymph and bone marrow. Liquid cancer is selected from the non-limiting group comprising leukemia, myeloma, myelodysplastic syndrome (MDS), and liquid lymphomas. For example, liquid cancer can be acute myeloid leukemia (AML). Liquid lymphomas include lymphomas that contain cysts or liquid areas.

Where the disease to be treated is an autoimmune disease, it is selected from the non-limiting group comprising rheumatoid arthritis (RA), multiple sclerosis (MS), endometriosis, inflammatory bowel disease (IBD), psoriasis, and psoriatic arthritis, or a combination of one or more thereof.

The polypeptides disclosed herein, or nucleic acids encoding such, may be introduced into the host cells using transfection and/or transduction techniques known in the art. The nucleic acid may be integrated into the host cell DNA or may be maintained extrachromosomally. The nucleic acid may be maintained transiently or may be a stable introduction. Transfection may be accomplished by a variety of means known in the art including but not limited to calcium phosphate-DNA co-precipitation, DEAE-dextran-mediated transfection, polybrene-mediated transfection, electroporation, microinjection, liposome fusion, lipofection, protoplast fusion, retroviral infection, and biolistics. Transduction refers to the delivery of a gene(s) using a viral or retroviral vector by means of viral infection rather than by transfection. In certain embodiments, retroviral vectors are transduced by packaging the vectors into virions prior to contact with a cell. For example, a nucleic acid encoding a transmembrane polypeptide carried by a retroviral vector can be transduced into a cell through infection and pro virus integration.

In certain aspects, the nucleic acid or vector, e.g. viral vector, is transferred via ex vivo transformation. Methods for transfecting cells and tissues removed from an organism in an ex vivo setting are known to those of skill in the art. Thus, it is contemplated that cells (or tissues) may be removed and transfected ex vivo using the polynucleotides presented herein. In particular aspects, the transplanted cells or tissues may be placed into an organism. Thus, it is well within the knowledge of one skilled in the art to isolate antigen-presenting cells ( e.g., T-cells or NK cells) from an animal ( e.g., human), transfect the cells with the expression vector and then administer the transfected or transformed cells back to the animal (e.g. human).

In certain aspects, the nucleic acid or vector is transferred via injection. In certain aspects, a polynucleotide is introduced into an organelle, a cell, a tissue or an organism via electroporation. In certain aspects, a polynucleotide is delivered into a cell using DEAE-dextran followed by polyethylene glycol.

In one aspect the method of treatment and/or prevention of a cancer, an infectious disease, an inflammatory or inflammation-induced disease, a chronic disease or an autoimmune disease in a subject comprises administering a pharmaceutical composition of the invention to a subject in need thereof.

In one aspect, the methods of treatment and/or prevention described above, can further comprise administrating at least one additional therapeutic agent or therapy.

In one aspect, the at least one additional therapeutic agent or therapy will be an anticancer agent or anticancer therapy, more preferably a therapeutically effective amount or dose of an anticancer agent or anticancer therapy. Said one or more anti-cancer agent or therapy will be selected among the non-limiting group comprising radiotherapy, chemotherapy, immune checkpoint inhibitor, immunotherapy and hormone therapy, or a combination of one of more thereof, as described herein.

In another aspect, the additional therapeutic agent can be an anti-inflammatory agent, as described herein.

The invention also contemplates kits for the treatment and/or prevention of a disease of the invention. In one aspect of the invention, the kit comprises a pharmaceutical composition of the invention.

The kits of the invention may also comprise a container and a label or package insert on or associated with the container. Suitable containers include, for example, bottles, vials, syringes, etc. The containers may be formed from a variety of materials such as glass or plastic. The container holds a composition which is effective for treating the disease of disorder of the invention and may have a sterile access port (for example the container may be an intravenous solution bag or a vial having a stopper pierceable by a hypodermic injection needle). Alternatively, or additionally, the kits may further comprise a second (or third) container comprising a pharmaceutically-acceptable buffer (such as bacteriostatic water for injection (BWFI), phosphate-buffered saline, Ringer's solution and dextrose solution) and/or at least one additional therapeutic agent (such as e.g. a ligand of the invention) . It may further include other materials desirable from a commercial and user standpoint, including other buffers, diluents, filters, needles, and syringes.

The label or package insert may comprise instructions for use thereof. Instructions included may be affixed to packaging material or may be included as a package insert. While the instructions are typically written or printed materials they are not limited to such. Any medium capable of storing such instructions and communicating them to an end user is contemplated by this disclosure.

The practice of the present disclosure will employ, unless otherwise indicated, conventional methods of protein chemistry, biochemistry, recombinant DNA techniques and pharmacology, within the skill of the art. Such techniques are explained fully in the literature. See, e.g., T. E. Creighton, Proteins: Structures and Molecular Properties (W.H. Freeman and Company, 1993); A. L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); REMINGTON'S PHARMACEUTICAL SCIENCES (Mack Pub. Co., N.J. 1991); Carey and Sundberg Advanced Organic Chemistry 3.sup.rd Ed. (Plenum Press) Vols A and B (1992).

The present disclosure is therefore to be considered as in all aspects illustrated and not restrictive, the scope of the invention being indicated by the appended Claims, and all changes which come within the meaning and range of equivalency are intended to be embraced therein.

### SEQUENCES

SEQ ID NO: 1 - mouse IL-4
   Amino acid sequence 
MGLNPQLVVILLFFLECTRSHIHGCDKNHLREIIGILNEVTGEGTPCTEMDVPNVLTATKNT
TESELVCRASKVLRIFYLKHGKTPCLKKNSSVLMELQRLFRAFRCLDSSISCTMNESKSTSL
KDFLESLKSIMQMDYS
SEQ ID NO: 2 - human IL-4
   Amino acid sequence
SEQ ID NO: 3 - HER2 scFv
   Amino acid sequence 
DYKDIVMTQSPSSLSASVGDRVTITCRASQDVNTAVAWYQQKPGKAPKLLIYSASFLYSGVP
SRFSGSRSGTDFTLTISSLQPEDFATYYCQQHYTTPPTFGQGTKVELKRATPSHNSHQVPSA
GGPTANSGEVKLVESGGGLVQPGGSLRLSCATSGFNIKDTYIHWVRQAPGKGLEWVARIYPT
NGYTRYADSVKGRFTISADTSKNTAYLQMNSLRAEDTAVYYCSRWGGDGFYAMDYWGQGTTV
TVSS
SEQ ID NO: 4 - TRP-1 scFv
   Amino acid sequence 
EVQLQQSGAELVRPGALVKLSCKTSGFNIKDYFLHWVRQRPDQGLEWIGWINPDNGNTVYDP
KFQGTASLTADTSSNTVYLQLSGLTSEDTAVYFCTRRDYTYEKAALDYWGQGASVIVSSGGG
GSGGGGSGGGGSDIQMSQSPASLSASVGETVTITCRASGNIYNYLAWYQQKQGKSPHLLVYD
AKTLADGVPSRFSGSGSGTQYSLKISSLQTEDSGNYYCQHFWSLPFTFGSGTKLEIKRA
SEQ ID NO: 5 - EGFRvIII scFv
   Amino acid sequence 
EVQVLESGGGLVQPGGSLRLSCAASGFTFSSYAMSWVRQAPGKGLEWVSAISGSGGSTNYAD
SVKGRFTISRDNSKNTLYLQMNSLRAEDTAVYYCAGSSGWSEYWGQGTLVTVSSGGGGSGGG
 
GSGGGGSDIQMTQSPSSLSASVGDRVTITCRASQGIRNNLAWYQQKPGKAPKRLIYAASNLQ
SGVPSRFTGSGSGTEFTLIVSSLQPEDFATYYCLQHHSYPLTSGGGTKVEIK
SEQ ID NO: 6 - CD19 scFv
   Amino acid sequence 
DIQMTQTTSSLSASLGDRVTISCRASQDISKYLNWYQQKPDGTVKLLIYHTSRLHSGVPSRF
SGSGSGTDYSLTISNLEQEDIATYFCQQGNTLPYTFGGGTKLEITGGGGSGGGGSGGGGSEV
KLQESGPGLVAPSQSLSVTCTVSGVSLPDYGVSWIRQPPRKGLEWLGVIWGSETTYYNSALK
SRLTIIKDNSKSQVFLKMNSLQTDDTAIYYCAKHYYYGGSYAMDYWGQGTSVTVSS
SEQ ID NO: 7 - CD8 transmembrane domain and hinge
   Amino acid sequence 
TTTKPVLRTPSPVHPTGTSQPQRPEDCRPRGSVKGTGLDFACDIYIWAPLAGICVALLLSLI
ITLICYHRSR
SEQ ID NO: 8 - CD3ζ
   Amino acid sequence 
RAKFSRSAETAANLQDPNQLYNELNLGRREEYDVLEKKRARDPEMGGKQQRRRNPQEGVYNA
LQKDKMAEAYSEIGTKGERRRGKGHDGLYQGLSTATKDTYDALHMQTLAPR
SEQ ID NO: 9 - 4-1BB
   Amino acid sequence
   KWIRKKFPHIFKQPFKKTTGAAQEEDACSCRCPQEEEGGGGGYEL

### EXAMPLES

### 1. Materials and Methods

**Mice.** Six to eight-week old female C57BL/6 (C57BL/6J) mice, and BALB/c (BALB/cByJ) mice were purchased from Charles River Laboratories (Lyon, France), maintained in École Polytechnique Fédérale de Lausanne (EPFL)-Center of PhenoGenomics (CPG) animal facility. Experimental procedures in mouse studies were approved by the Swiss authorities (Canton of Vaud, animal protocol ID 3533) and performed in accordance with the guidelines from CPG of EPFL, the animal facility of University of Lausanne.

**Cells and tumor models.** HER2 transduced MC38 mouse colon cancer cells (MC38-HER2) were generated according to the previous publications^{18,19}. B16F10 melanoma cell and Phoenix-Eco cell were originally acquired from the American Type Culture Collection. 4T1-EGFRvIII-Luc mouse breast cancer cells were provided by Prof. Darrell J. Irvine (Massachusetts Institute of Technology). All the mouse tumor cells were cultured in complete Dulbecco's modified Eagle's medium (DMEM), a DMEM (Gibco/Thermo Fisher Scientific) medium supplemented with fetal bovine serum (FBS) (10% v/v, Gibco/Thermo Fisher Scientific), and penicillin/streptomycin (1% v/v, Gibco/Thermo Fisher Scientific). MC38-HER2 (3 × 10⁵) and B16F10 tumor cells (3 × 10⁵) were implanted subcutaneously (s.c.) into the right flanks of C57BL/6 wild-type (WT) mice to establish the syngeneic tumor models. 4T-EGFRvIII-Luc tumor cells (5 × 10⁴) were injected intravenously (i.v.) into BALB/c WT mice.

### Construction of CARs

CAR constructs targeting HER2 were generated as previously described²⁰. Briefly, the complete CAR sequence is composed of a mouse CD8 signal peptide, antigen-specific scFv (TA99 scFv for monospecific TRP-1 CAR, 139scFv for EGFRvIII CAR), mouse CD8a hinge, and transmembrane domain, 4-1BB costimulatory domain, and CD3ζ intracellular domain. To facilitate CAR detection by flow cytometry, a c-Myc tag was inserted either between the scFv and CD8a hinge for the EGFRvIII CAR and TRP-1 CAR. The full-length mouse IL-4 gene (accession number NP_034678.1) was amplified by PCR from the cDNA clone custom purchased from Twist Bioscience, respectively. IL-4 CAR constructs were generated by fusing 2A self-cleaving peptide and IL-4 gene fragments into the CAR containing viral vector (pMSGV for HER2 CAR, pMSCV for TRP-1 CAR and EGFRvIII CAR).

**Retrovirus production.** Retrovirus production was carried out using the calcium phosphate method following the manufacturer's protocol (Clonetech). Briefly, phoenix cells were seeded in a 10 cm dish and cultured for 18 h before transfection and replenished with 10 ml of pre-warmed medium without disturbing the cells before transduction. For each transfection, 14 µg of plasmid (8.5 µg of CAR plasmid plus 5.5 µg of pCL-Eco packaging plasmid) was added to 628 µl of ultrapure water, followed by addition of 72 µl of a CaCl₂ solution (2 M, Sigma-Aldrich). Hepes Buffered Saline (700 µl × 2, Sigma-Aldrich) was then added in a dropwise manner with gentle vortexing. After a 20-min incubation at 25°C, the transfection mixture was gently added to phoenix cells, and replenished with 10-ml pre-warmed medium 16 h later. After 36-72 h incubation, virus-containing supernatant was collected and passed through a 0.45-µm filter (Merck Millipore) to remove cell debris. Virus-containing supernatant was then aliquoted and stored at -80°C.

**Lentivirus production.** Lentivirus production was carried out using the polyethylenimine (PEI) method following the manufacturer's protocol (Polysciences, Inc.). Briefly, 1 × 10⁷ Human Embryonic Kidney (HEK) cells were seeded in a T150 cell culture flask and cultured for 24 hours before transfection. For each transfection, 15 µg of CAR plasmid, 18 µg of Δ8.9 plasmid, and 7 µg of VSV-G plasmid was added to 2 mL of serum-free media, followed by addition of 100 µg of PEI. The transfection mixture was incubated at 25°C for 20 minutes, during which the media from the HEK cells was aspirated. The mixture was then gently added to the HEK cells and swirled around. 14mL of serum-containing media was added to the HEK cells, then the flask was placed in the incubator at 37°C for 2 days. After 48 hours of incubation, virus-containing supernatant was collected and passed through a 0.45-µm filter (Merck Millipore) to remove cell debris. Virus-containing supernatant was then aliquoted and stored at -80°C.

**Preparation of mouse CAR-T cells.** Spleens from WT mice were disintegrated mechanically and filtered through a 70-µm strainer (Fisher Scientific). Red blood cells (RBC) were lysed with ACK lysis buffer (2 ml per spleen, Gibco/Thermo Fisher Scientific) for 5 min at 25°C. The splenocytes were washed once with cold complete RPMI medium, which contained RPMI-1640 (Gibco), FBS (10% v/v, Gibco/Thermo Fisher Scientific), HEPES (1% v/v, Gibco/Thermo Fisher Scientific), penicillin/streptomycin (1% v/v, Gibco/Thermo Fisher Scientific), sodium pyruvate (1% v/v, Gibco/Thermo Fisher Scientific), and 2-mercaptoethanol (0.1% v/v, Gibco / Thermo Fisher Scientific), were then resuspended at a cell density of 2 × 10⁶ cells/ml in a complete RPMI medium supplemented with IL-2 (10 ng/ml, PeproTech) and IL-7 (10 ng/ml, PeproTech). For WT T cell activation, 6-well plates were pre-coated with 1 ml of anti-CD3 (1 µg/ml, 17A2, BioXCell) and anti-CD28 (5 µg/ml, 37.51, BioXCell) per well at 4°C for 18 h. T cells were enriched by using Ficoll-Paque PLUS (GE Healthcare) and seeded onto pre-coated 6-well plates at 3 × 10⁶ cells/well in complete RPMI medium (3 ml per well). Cells were cultured at 37°C for 48 h without disturbance. 6-well plates were coated with protamine (10 µg/ml, Sigma-Aldrich) for 24 h prior to transduction. On day 2, protamine-coated plates were blocked with FBS (0.5% v/v) containing PBS for 30 min before use. Virus supernatant was first added into each well of the blocked plates (1 ml per well), plates were centrifuged at 2000g for 2 h at 32°C. Activated T cells were collected, enriched again by using Ficoll-Paque PLUS and resuspended at 2 × 10⁶ cells/ml in a complete RPMI medium supplemented with IL-2 (10 ng/ml). Above cell suspension was added virus enriched plates (1 ml per well) and mixed well by gentle shaking, spin transfection was carried out at 500g for 30 min at 32°C. Plates were then transferred to an incubator and maintained overnight. A second transduction was conducted on day 3, T cells were collected and added into virus containing plate post centrifugation. After overnight incubation, T cells were collected, and expanded in a fresh complete RPMI medium supplemented with IL-2 (10 ng/ml). On day 5, transduction efficiency was determined by flow cytometry analyses. For HER2 CAR-T, CAR expression was detected by using a biotinylated human Her2/ErbB2 Protein (Aero Biosystems) and the secondary staining reagent Streptavidin-PE/Cyanine7 (BioLegend). TRP-1 CAR or EGFRvIII CAR expression was evaluated by surface staining of c-Myc tag using an anti-Myc antibody (9B11, Cell signaling). Untransduced T cells activated and cultured in parallel with virus-free medium were used as control. CAR-T cells were used on day 5 for both in vitro and in vivo experiments. IL-4 concentration in the culture supernatants was determined by ELISA MAX^{™} Standard Set Mouse IL-4 (BioLegend) post 2-day coculture after CAR-T generation.

**Preparation of human CAR-T cells.** Human peripheral blood mononuclear cells were obtained from anonymous healthy donors (prepared as buffy coats). CD3+ T cells were isolated using the Pan T Cell Isolation Kit, human from Miltenyi Biotec (cat#: 130-096-535). The CD3+ T cells were activated in vitro with Dynabeads^{™} Human T-Activator CD3/CD28 for T Cell Expansion and Activation (cat#: 11161D), in the presence of human IL-2 (50 IU/mL) for 5 days. On day 1, T cells were placed in 24 well plates at 2mL per plate at a concentration of 1 × 10⁶ cells/mL. 1.4 mL of media was removed, afterwhich lentivirus was added to the culture plates at an MOI of 2-3, in the presence of 20ug/mL of protamine. Human IL-2 was added to reach a total concentration of 30 IU/mL. The cells were centrifuged for 30 minutes at 800 × g in 32°C. The cells were incubated at 37°C for 4 hours, afterwhich 1mL of pre-warmed R10 media with human IL-2 (30IU/mL) were added to each well and the cells were incubated at 37°C for 48 hours. On day 5, the Dynabeads^{™} were removed using a magnetic cell separation. On day 10, transduction efficiency was determined by flow cytometry analyses. For CD19 CAR-T, CAR expression was detected by using monoclonal anti-FMC63 antibody, Mouse IgG1 (Y45) from Aero Biosystems (cat#: FM3-Y45). Nontransduced T cells (NTD) were activated and cultured in parallel and virus-free medium was used as control. CAR-T cells were used on day 10 for in vitro. IL-4 concentration in the culture supernatants was determined by ELISA MAX^{™} Standard Set Mouse IL-4 (BioLegend) post 2-day coculture after CAR-T generation.

**Flow cytometry analyses.** For surface marker staining, cells were collected into U-bottom 96-well plates (Thermal Fischer Scientific), blocked with anti-mouse CD16/32 antibody (BioLegend), and incubated with indicated antibodies at 4 °C for 20 min, followed by live/dead staining by 4',6-diamidino-2-phenylindole (DAPI, Sigma-Aldrich). Cells were then washed and resuspended with PBS containing bovine serum albumin (BSA, 0.2% wt/v, Sigma-Aldrich) for flow cytometry analyses. Afterward, cells were further stained for surface markers and DAPI for flow cytometry analyses. For intracellular cytokine staining, cells were first stimulated by a Cell Stimulation Cocktail (Invitrogen/Thermo Fisher Scientific) at 37°C for 5 h. After stimulation, cells were first stained for surface markers and Zombie Aqua Fixable Dye (BioLegend), then fixed and permeabilized with a Cytofix/Cytoperm^{™} Fixation/Permeabilization Solution Kit (BD Biosciences). Intracellular staining with indicated antibodies was performed following the manufacturer's protocol. Cells were detected using an Attune NxT Flow Cytometer with Attune NxT Software v.3 (Invitrogen/Thermal Fischer Scientific). Analyses were performed using FlowJo 10.6.1 (Tree Star). Gate margins were determined by isotype controls, and fluorescence-minus-one controls.

**Antibodies and reagents for flow cytometry.** The following antibodies or staining reagents were purchased from BioLegend: CD16/32 (93, 101302), CD45.2 (104, 109814), CD8β (YTS256.7.7, 126606), CD4 (RM4-5, 100526), CD3ε (17A2, 100306), CD11c (N418, 117348), I-A/I-E (MHC-II, M5/114.15.2, 107643), Granzyme B (GB11, 515403), IFNγ (XMG1.2, 505826), TNFα (MP6-XT22, 506308), IL-4 (11B11, 504133), Streptavidin-PE/Cyanine7 (405206). Biotinylated Human Her2/ErbB2 Protein (HE2-H822R) was purchased from ACROBiosystems. Myc-tag antibody (9B11, 3739) was obtained from Cell Signaling Technology.

**Antitumor therapy and rechallenging experiments.** C57BL/6 or BALB/c WT bearing established tumors were sublethally lymphodepleted by total body irradiation (5 Gy) on day 5. On day 6, mice received i.v. adoptive transfer of CAR-T cells (HER2 CAR-T, TRP-1 CAR-T, or EGFRvIII CAR-T, 3 × 10⁶ CAR⁺ T cells) in the presence or absence of i.v. administered IL-4 (1 µg), IL-4 CAR-T cells (IL-4 HER2 CAR-T, IL-4 TRP-1 CAR-T, or IL-4 EGFRvIII CAR-T, 3 × 10⁶ CAR⁺ T cells), or PBS. For the MC38-HER2 or B16F10 subcutaneous model in C57BL/6 mice, tumor area and body weight were measured every other day. Tumor area was calculated by the formula Area = Length × Width from caliper measurements of 2 orthogonal diameters. For the 4T1-EGFRvIII-Luc metastatic tumor model, BALB/c mice were anesthetized and intraperitoneally injected with bioluminescent substrate D-Luciferin potassium salt (100 µl, GoldBio) prediluted at 30 mg/ml in 1 × PBS. Ten minutes post injection, mice were subjected to luminescent imaging using a Xenogen IVIS fluorescence/bioluminescence imaging system for tumor growth monitoring. In tumor cell rechallenging experiments, MC38-HER2 (1 × 10⁶), B16F10 (1 × 10⁵) cells were re-inoculated s.c. into the left flanks of survived mice from treatment groups 3 months post adoptive CAR-T cell transfer. Naive WT mice were s.c. inoculated with the same number of tumor cells as control. Survivor of re-challenged mice was monitored for at least another 60 days. Mice were euthanized when body weight loss was beyond 15% of baseline weight, tumor area reached 150 mm², or any signs of discomfort were detected by the investigators or as recommended by the veterinarian who monitored the mice every other day.

**Analyses of tumor-infiltrating immune cells.** C57BL/6 mice were inoculated s.c. with MC38-HER2 tumor cells (1 × 10⁶), sublethally lymphodepleted by irradiation on day 5, and received i.v. adoptive transfer of HER2 CAR-T cells (3 × 10⁶ CAR⁺ T cells) in the presence or absence of i.v. administered IL-4 (1 µg), IL-4 HER2 CAR-T (3 × 10⁶ CAR⁺ T cells), or PBS control on day 6 post tumor inoculation. On day 14, tumors were collected, weighed, mechanically minced, and digested in RPMI-1640 medium supplemented with collagenase Type IV (1 mg/ml, Gibco/Thermo Fisher Scientific), dispase II (100 µg/ml, Sigma-Aldrich), hyalurondase (100 µg/ml, Sigma-Aldrich), and DNase I (100 µg/ml, Sigma-Aldrich) at 37 °C for 60 min. RBC lysis was performed on the digested tumor samples with ACK lysing buffer. Tumor-infiltrating leukocytes were then enriched by Percoll (GE healthcare) density gradient centrifugation, resuspended in PBS with BSA (0.2%, wt/v), stained with indicated antibodies, and analyzed by flow cytometry.

**Analyses** of CAR-T cells from spleen. C57BL/6 or BALB/c WT bearing established tumors were sublethally lymphodepleted by total body irradiation (5 Gy) on day 5. On day 6, mice received i.v. adoptive transfer of CAR-T cells (HER2 CAR-T, or EGFRvIII CAR-T, 3 × 10⁶ CAR⁺ T cells), IL-4 CAR-T cells (IL-4 HER2 CAR-T, or IL-4 EGFRvIII CAR-T, 3 × 10⁶ CAR⁺ T cells), or PBS. Spleens were collected for analyses of HER2 CAR-T and EGFRvIII CAR-T cells on day 14 and day 18, respectively. Spleens were dissected from the surrounding tissues, grinned, and filtered through a 70-µm strainer (Fisher Scientific). RBC lysis was performed on the spleen samples with ACK Lysing Buffer (2 ml per spleen, Gibco/Thermo Fisher Scientific) and then resuspended in PBS with BSA (0.2%, wt/v). RBCs were lysed by ACK lysis buffer, then debris of RBCs was removed by Percoll (GE healthcare), and then resuspended in PBS with BSA (0.2%, wt/v). Collected cells from the above samples were stained with indicated antibodies and analyzed by flow cytometry.

***In vitro* coculture of CAR-T cells and tumor cells.** MC38-HER2, B16F10, 4T1-EGFRvIII-Luc, and NALM6-GFP tumor cells (1 × 10⁵/well) were seeded in 12-well plates (Thermal Fischer Scientific) in a complete DMEM medium and incubated overnight. After aspiration of tumor culture medium, HER2 CAR-T, TRP-1 CAR-T, EGFRvIII CAR-T, or human CD19 CAR-T cells on day 5 (mouse) or day 10 (human) as described above were suspended in complete RPMI medium supplemented with IL-2 (10 ng/ml) (mouse) or human IL-2 (30 IU/mL) (human) and added to the tumor cell culture at the E:T ratio of 0.5 to 1 in the presence or absence of IL-4 (145ng/ml) for mouse, and E:T ratio of 1:5. After 1-day coculture, cells in the plates were collected, and conducted flow cytometry analyses to determine the cytotoxicity phenotype of CAR-T cells. After 2-day coculture, cells in the plates were collected, and conducted flow cytometry analyses to determine the viability of tumor cells, proliferation, and exhaustion phenotype of CAR-T cells.

**Seahorse assay.** MC38-HER2 tumor cells (1 × 10⁶/flask) were seeded in T25 flasks (Thermal Fischer Scientific) in complete DMEM medium and incubated overnight. After aspiration of tumor cell culture medium, HER2 CAR-T or IL-4 HER2 CAR-T cells in complete RPMI medium supplemented with IL-2 (10 ng/ml) were added to the tumor cell culture at an E:T ratio of 5 to 1. After 16-h coculture, CAR-T cells in the flasks were collected and isolated by Ficoll-Paque PLUS for seahorse assay. Seahorse assay was performed to measure oxygen consumption rate (OCR) and extracellular acidification rate (ECAR) of CAR-T cells. CAR-T cells (3 × 10⁵/well) in normal culture condition or post coculture were seeded in a seahorse culture plate (Seahorse Bioscience) in non-CO₂ incubator at 37 °C for 40 min. OCR and ECAR were measured by a Seahorse XFe96 Analyzers (Seahorse Bioscience) following the manufacturer's instructions. During a seahorse assay, cells were treated with oligomycin (1 µM, Sigma-Aldrich), Carbonyl cyanide-4-(trifluoromethoxy)phenylhydrazone (FCCP, 2 µM, Sigma-Aldrich), rotenone (0.5 µM, Sigma-Aldrich), antimycin A (0.5 µM, Sigma-Aldrich), glucose (10 mM, Sigma-Aldrich), and 2-Deoxy-D-glucose (2-DG, 50 mM, Sigma-Aldrich). Each condition was performed with 3-6 replicates in a single experiment. Basal or maximal OCR and ECAR were calculated according to previous reports²¹.

**Metabolic inhibitor treatments.** MC38-HER2 cells (1 × 10⁵/well) were seeded on 12-well plates (Thermal Fischer Scientific) and incubated overnight. HER2 CAR-T or IL-4 HER2 CAR-T cells on day 6 as described above were cocultured with pre-seeded MC38-HER2 cells at an E:T ratio of 0.5 to 1 in complete RPMI medium containing IL-2 (10 ng/ml) and indicated inhibitors for 2 days. Phenotypes of CAR-T cells were determined by flow cytometry analyses.

**Statistical analysis**. Statistical analysis was performed using GraphPad Prism 9 (GraphPad software). Data are presented as mean ± s.e.m. unless otherwise indicated. Comparisons of two groups were performed by using two-tailed unpaired Student's t test. Comparisons of multiple groups were performed by using one or two-way analysis of variance (ANOVA) with Tukey's multiple-comparisons test. Survival data were analyzed using the Log-rank test. No statistically significant (NS) differences were considered when P-values were larger than 0.05.

### 2. Results

2.1 We hypothesized that CAR-T cells armored with type 2 cytokine can enhance cytotoxicity of CAR-T cells and stimulate immune activation that would counter immunosuppression of tumor microenvironment (TME) in solid tumors. To test this hypothesis, we first generated murine IL-4-expressing CAR-T cells-based on the conventional second-generation CAR, HER2 scFv-CD8 transmembrane (TM)-4-1BB-CD3 zeta (Fig. 1a). Cell surface expression of HER2 CAR in IL-4 HER2 CAR-T cells was almost equivalent to or slightly lower than that in conventional HER2 CAR-T cells (**Fig. 1b**). HER2 CAR-T cells engineered to express IL-4 produced IL-4 during *in vitro* culture (Fig. 1c). IL-4-expressing HER2 CAR-T cells showed higher tumor-lytic potential against MC38-HER2 cells as compared to HER2 CAR-T cells (Fig. 1d).

To predict *in vivo* activity in the TME, we cocultured MC38-HER2 cells and HER2 CAR-T cells with or without IL-4 expression at low E:T ratios for 2 days. In the coculture with MC38-HER2 cells, IL-4 HER2 CAR-T cells exhibited greatly enhanced proliferation, polyfunctionality, as w ell as killing efficiency of target cells **(****Fig. 1e****-g),** which were consistent with the tumor lysis assay we have shown above.

2.2 We next assessed whether IL-4 expression could reprogram CAR-T cell metabolism. IL-4-expressing HER2 CAR-T cells exhibited markedly elevated basal and maximal ECAR as compared to HER2 CAR-T cells, whereas OCR remained almost unchanged **(****Fig. 1h****-k).** This finding was consistently showed when there is persistent tumor antigen stimulation (**Fig. 1l-o**)**.** These results indicate that IL-4 expression reprograms CAR-T cell metabolism toward glycolysis, which is antigen independent.

2.3 We next used several pathway specific inhibitors to probe the molecular basis of metabolic regulation of CAR-T cells by secreted IL-4. By apply specific inhibitors for glycolysis related metabolic pathways, we found inhibition pyruvate generation from glucose by 2-Deoxy-D-glucose (2-DG), 3-Bromopyruvate (3BP), 6-aminonicotinamide (6-AN), and sodium fluoride (NaF) did not impair the IL-4 induced cytotoxic CAR-T cell proliferation **(****Fig. 1p**). Notably, inhibition of lactate dehydrogenase A (LDHA) by FX11 significantly abrogated the IL-4-mediated enrichment of cytotoxic CAR-T cells **(****Fig. 1p****).** Given that LDHA plays a central role on the conversion of pyruvate to lactate, our results suggest IL-4 expression enhance effector function of CAR-T cells by enhancing glycolysis in a LDHA-dependent manner.

2.4 We next examined whether HER2 CAR-T cells armored with IL-4 could counter dysfunction and sustain the effector function in tumors. We i.v. transferred IL-4 HER2 CAR-T cells (3 × 10⁶) or HER2 CAR-T cells (3 × 10⁶) in the presence or absence of i.v. administered free mouse IL-4 to treat established s.c. MC38-HER2 murine colon adenocarcinoma tumors in a mouse model. In comparison to the conventional CAR-T cells, IL-4 HER2 CAR-T cells exhibited ~4.1-fold increase of cell counts in the tumor, suggesting IL-4 expression greatly promotes CAR-T cell proliferation in TME **(****Fig. 2a****).** Moreover, IL-4 HER2 CAR-T cells also exhibited significantly increased cell counts as compared with conventional HER2 CAR-T cells in secondary lymphoid organs, including spleen and tumor draining lymph node **(****Fig. 2b, c****).** IL-4 expression promoting TIM-3^{high} PD-1^{high} CAR-T cells differentiation towards TIM-3^{high} PD-1^{low} phenotype subset without changing progenitor exhausted subset **(****Fig. 2d****-f).**

Importantly, IL-4 HER2 CAR-T cells also showed enhanced cytotoxicity and polyfunction, but reduced expression of inhibitory marker PD-1 **(****Fig. 2g****-i).** As IL-4 is a type 2 cytokine that may repolarize T cells to promote a strong type 2 immunity, we next investigated whether IL-4-expressing CAR-T cells alter T cells subset compartment. We observed IL-4-expressing HER2 CAR-T cells enrich in Th2 cells and type 2 cytotoxic (Tc2) cells among CAR-T cells in tumors **(****Fig. 2j, k**). These results suggest that IL-4-expressing CAR-T cells possess distinguished antitumor features compared to conventional CAR-T cells, including hyperproliferative capacity, enhanced cytotoxicity, and activated type 2 T cell populations.

2.5 The finding that IL-4 HER2 CAR-T cells enhanced expansion and effector function motivated us to assess their efficacy against solid tumors. To treat the preestablished MC38-HER2 subcutaneous solid tumors in mice, we transferred IL-4 HER2 CAR-T cells (3 × 10⁶) through i.v. administration, which led to 40% of tumor clearance and curative responses **(****Fig. 3a, b**). To investigate whether IL-4-expressing HER2 CAR-T cells developed antitumor immune memory, we rechallenged the surviving mice 3 months post adoptive CAR-T cell transfer. Impressively, 100 % of long-term survivors treated with IL-4 HER2 CAR-T cells rejected a second challenge of the original tumor cells **(****Fig. 3c****).** As the one of the major causes of treatment failure of CAR-T cells against solid tumors is antigen downregulation or loss. We thus next evaluated whether IL-4-expressing CAR-T cell resist the challenge of antigen negative MC38 tumors in long-term surviving mice post rechallenge of MC38-HER2 cells **(****Fig. 3d****).** Interestingly, we found the survivors of IL-4-expressing CAR-T cells not only rejected HER2 positive tumor growth but also completely resist the challenge of HER2 negative tumors **(****Fig. 3d****).** This discovery motivated us to determine whether endogenous cell types contribute to IL-4-CAR-T cells induced antitumor responses in rejecting antigen negative tumors. To identify changes of immune cells in tumors, we performed flowcytometry analysis on tumor-infiltrating cells purified from HER2 CAR-T cells treated MC38-HER2 tumor bearing mice. Compared with HER2 CAR-T cells, IL-4 HER2 CAR-T cell treatment significantly increased number of CD45.2⁺ cells and led to an enrichment in mature DCs in tumors **(****Fig. 3e, f**). Together, these changes appear to promote antigen negative tumor control of IL-4-expressing CAR-T cells, suggesting that IL-4 may have pleiotropic effects to shift the balance of immune populations toward durable antitumor immunity against both antigen positive and pre-existing antigen negative tumor cells.

2.6 We next extended this strategy to the CAR specific to melanoma-associated antigen TRP-1 and prepared IL-4 TRP-1 CAR-T cells **(****Fig. 4a****-c).** IL-4 TRP-1 CAR-T cells exhibited enhanced expansion and killing efficiency of TRP-1-expressing B16F10 melanoma cells in vitro compared to conventional TRP-1 CAR-T cells **(****Fig. 4d, e**). In a therapeutic setting with the poorly immunogenic and highly aggressive mouse B16F10 melanoma in mice with lymphodepletion preconditioning, adoptive transfer of IL-4 TRP-1 CAR-T cells (3 × 10⁶) induced complete tumor remission and durable cures in 50% of treated mice **(****Fig. 4f, g**). By contrast, adoptive transfer of TRP-1 CAR-T cells alone or combined with i.v. administration of IL-4 failed to induce any durable tumor regression **(****Fig. 4f, g**). We next evaluated whether IL-4-expressing CAR-T cells could control metastatic tumor. We prepared EGFRvIII-targeting CAR-T cells with IL-4 expression **(****Fig. 5a****-c).** IL-4 EGFRvIII CAR-T cells exhibited enhanced proliferation and in vitro killing efficiency of 4T1-EGFRvIII-Luc target cells as compared to EGFRvIII CAR-T cells **(****Fig. 5d, e**). To establish a metastatic tumor model, BALB/c mice received i.v. injection of 4T1-EGFRvIII-Luc cells. The mice that developed metastatic tumors in the lungs were sub lethally lymphodepleted and then received the adoptive transfer of EGFRvIII CAR-T cells alone or in combination with free mouse IL-4 infusion or IL-4-expressing EGFRvIII CAR-T cells. The tumor growth was monitored by bioluminescence imaging. Notably, IL-4 EGFRvIII CAR-T cells exhibited superior anti-metastases activity and led to durable cures in 20% of treated mice, whereas EGFRvIII CAR-T cells with or without exogenous IL-4 infusion showed only modest tumor burden control **(****Fig. 5f, g**). Collectively, these results suggest that IL-4-expressing CAR-T cells represent a potent immunotherapy against solid tumors.

In summary, we have shown that CAR-T cells engineered to express IL-4 exhibited enhanced proliferative capacity, effector function, and prevented the functional impairment in solid tumors. Metabolic analyses revealed that IL-4 expression promoted glycolysis of CAR-T cells in a LDHA-dependent manner. Adoptive transfer of IL-4-expressing CAR-T cells eradicated established solid tumors in three syngeneic mouse models, including metastasis tumor model. Furthermore, IL-4 secretion in CAR-T cells induced maturation of DCs in tumors, which might contribute to a broad immune protection in treated mice against rechallenge of both antigen positive and negative tumors. The IL-4 armored CAR-T cell is a promising therapeutic strategy to retain T cell effector function and induce potent and broad antitumor immunity.

### REFERENCES

1. Lim, W. A. & June, C. H. The Principles of Engineering Immune Cells to Treat Cancer. Cell 168, 724-740 (2017).
2. Ye, L. et al. A genome-scale gain-of-function CRISPR screen in CD8 T cells identifies proline metabolism as a means to enhance CAR-T therapy. Cell Metab 34, 595-614.e14 (2022).
3. Sun, J. et al. T Cells Expressing Constitutively Active Akt Resist Multiple Tumor-associated Inhibitory Mechanisms. Molecular Therapy 18, 2006-2017 (2010).
4. Wu, Y., Deng, Z., Tang, Y., Zhang, S. & Zhang, Y.-Q. Over-expressing Akt in T cells to resist tumor immunosuppression and increase anti-tumor activity. BMC Cancer 15, 603 (2015).
5. Kawalekar, O. U. et al. Distinct Signaling of Coreceptors Regulates Specific Metabolism Pathways and Impacts Memory Development in CAR T Cells. Immunity 44, 380-390 (2016).
6. Rodolfo, M. et al. IL-4-Transduced Tumor Cell Vaccine Induces Immunoregulatory Type 2 CD8 T Lymphocytes That Cure Lung Metastases Upon Adoptive Transfer1. The Journal of Immunology 163, 1923-1928 (1999).
7. Golumbek, P. T. et al. Treatment of Established Renal Cancer by Tumor Cells Engineered to Secrete Interleukin-4. Science (1979) 254, 713-716 (1991).
8. Momin, N. et al. Anchoring of intratumorally administered cytokines to collagen safely potentiates systemic cancer immunotherapy. Sci Transl Med 11, eaaw2614 (2019).

## Claims

1. An immune cell expressing an interleukin-4, a fragment or a variant thereof, said immune cell comprising one or more recombinant constructs, wherein at least one recombinant construct encodes an interleukin-4 (IL-4), a fragment or a variant thereof.

2. The immune cell of claim 1, wherein a at least one recombinant construct encodes a chimeric antigen receptor (CAR), a T cell receptor (TCR) or any other synthetic tumor targeting motif.

3. The immune cell of claim 1 or 2, wherein the immune cell is a T cell, chimeric antigen receptor (CAR)-T cell, T cell receptor (TCR)-transgenic T cell, tumor infiltrating lymphocyte (TIL), NK cell, NK-T cell, CAR-NK cell, CAR-NKT cell, TCR-transgenic NK cell, TCR-transgenic NK-T cell, dendritic cell, macrophage, CAR-macrophage or any synthetic tumor specific immune cells.

4. The immune cell of any one of the preceding claims, wherein the construct encoding an IL-4, a fragment or a variant thereof encodes i) a sequence comprising, or consisting of, SEQ ID NO. 1, a variant or a functional fragment thereof or ii) a sequence comprising, or consisting of, SEQ ID NO. 2, a variant or a functional fragment thereof.

5. The immune cell of any one of the preceding claims, wherein the recombinant construct encoding an IL-4, a variant or a functional fragment thereof is linked to the second recombinant construct encoding a CAR, a TCR, or any other synthetic tumor targeting motif.

6. The immune cell of claim 5, wherein the recombinant construct encoding an IL-4, a variant or a functional fragment thereof is linked to the at least one recombinant construct encoding a CAR, a TCR or any other synthetic tumor targeting motif, via a sequence encoding a self-cleaving peptide (e.g. peptide 2A).

7. The immune cell of claim 5 or 6, wherein the at least one recombinant construct encoding the CAR comprises
- at least one antigen binding domain,
- a transmembrane domain,
- at least one costimulatory domain, and
- at least one activating domain.

8. The immune cell according to claim 7, wherein the at least one antigen binding domain is an extracellular antigen recognition domain of a single-chain Fragment variant (scFv) derived from an antibody recognizing an antigen selected from the group comprising HER2, TRP-1, EGFRvIII, , CD19, CD20, CD38, CD30, ERBB2, fibroblast activation protein (FAP), CA125, MUC-1, PSMA, PSA, CD44 surface adhesion molecule, mesothelin, carcinoembryonic antigen (CEA), CEACAM5, CEACAM6, epidermal growth factor receptor (EGFR), vascular endothelial growth factor receptor-2 (VEGFR2), high molecular weight-melanoma associated antigen (HMW-MAA), MAGE-A1, IL-13R-a2, GD2, carbonic anhydrase EX, alpha-fetoprotein, A3, antigen specific for A33 antibody, Ba 733, BrE3-antigen, CD1, CDIa, CD3, CD5, CD15, CD16, CD19, CD20, CD21, CD22, CD23, CD25, CD30, CD33, CD38, CD45, CD74, CD79a, CD80, CD138, colon-specific antigen-p (CSAp), CSAp, EGP-I, EGP-2, Ep-CAM, FIt-I, Flt-3, folate receptor, HLA-DR, human chorionic gonadotropin (HCG) and its subunits, hypoxia inducible factor (HIF-I), Ia, IL-2, IL-6, IL-8, insulin growth factor-1 (IGF-I), KC4-antigen, KS-1-antigen, KS1-4, Le-Y, macrophage inhibition factor (MIF), MAGE, MUC1, MUC2, MUC3, MUC4, NCA66, NCA95, NCA90, tyrosinase, PRAME, EBNA, KLK3, HPV E7, LMP2, NY-ESO-1, PAP, reverse transcriptase, nucleophosmin, PRTN3/ELANE, CT83/KKLC1, MUC16, DNTT, antigen specific for PAM-4 antibody, placental growth factor, p53, prostatic acid phosphatase, RS5, S1OO, TAC, TAG-72, tenascin, TRAIL receptors, Tn antigen, Thomson-Friedenreich antigens, tumor necrosis antigens, VEGF, ED-B fibronectin, 17-1A-antigen, NeuGcGM3, N-glycolyl GM3 ganglioside, Neu5Gc, GM3-Ganglioside, GD3, GM2, carbohydrate antigens, ganglioside antigens, Lewis Y, Lewis B, MOG, MBP, aB-crystallin, PLP, GlialCAM, β-synuclein, HLA-A2, TNP, CD123, Kappa chain of immunoglobulin, or any combination thereof.

9. The immune cell according to claim 7 or 8, wherein the transmembrane domain is selected from the group comprising CD28, CD28T, OX-40, 4-1BB/CD137, CD2, CD7, CD27, CD30, CD40, programmed death-1 (PD-1), inducible T cell costimulator (ICOS), CDl-la/CD18, CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD 162), LTBR, LAT, GADS, SLP-76, PAG/Cbp, CD 19a, a ligand that specifically binds with CD83, or any combination thereof.

10. The immune cell according to any one of claims 7 to 9, wherein the costimulatory domain is a signalling region (or other suitable portion) comprising CD28, OX-40, 4-1BB/CD137, CD2, CD7, CD27, CD30, CD40, programmed death-1 (PD-1), inducible T cell costimulator (ICOS), lymphocyte function-associated antigen-1 (LFA-1 (CDl la/CD18), CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class I molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD 19a, a ligand that specifically binds with CD83, or any combination thereof.

11. The immune cell according to any one of claims 7 to 10, wherein the at least one activating domain is a T cell activation domain, preferably a CD3 domain, more preferably a CD3 zeta (CD3ζ) domain.

12. A CAR polypeptide comprising
- at least one antigen binding domain,
- a transmembrane domain,
- at least one costimulatory domain,
- at least one activating domain, and
- at least one interleukin 4 (IL-4) molecule, a variant or a functional fragment thereof.

13. The CAR polypeptide according to claim 12, wherein the at least one IL-4 molecule comprises a sequence selected from the group comprising, or consisting of, i) SEQ ID NO. 1, a variant or a functional fragment thereof or ii) SEQ ID NO. 2, a variant or a functional fragment thereof.

14. The CAR polypeptide according to claim 12 or 13, wherein the at least one antigen binding domain is an extracellular antigen recognition domain of a single-chain Fragment variant (scFv) derived from an antibody recognizing an antigen selected from the group comprising HER2, TRP-1, EGFRvIII, , CD19, CD20, CD38, CD30, ERBB2, fibroblast activation protein (FAP), CA125, MUC-1, PSMA, PSA, CD44 surface adhesion molecule, mesothelin, carcinoembryonic antigen (CEA), CEACAM5, CEACAM6, epidermal growth factor receptor (EGFR), vascular endothelial growth factor receptor-2 (VEGFR2), high molecular weight-melanoma associated antigen (HMW-MAA), MAGE-A1, IL-13R-a2, GD2, carbonic anhydrase EX, alpha-fetoprotein, A3, antigen specific for A33 antibody, Ba 733, BrE3-antigen, CD1, CDIa, CD3, CD5, CD15, CD16, CD19, CD20, CD21, CD22, CD23, CD25, CD30, CD33, CD38, CD45, CD74, CD79a, CD80, CD138, colon-specific antigen-p (CSAp), CSAp, EGP-I, EGP-2, Ep-CAM, FIt-I, Flt-3, folate receptor, HLA-DR, human chorionic gonadotropin (HCG) and its subunits, hypoxia inducible factor (HIF-I), Ia, IL-2, IL-6, IL-8, insulin growth factor-1 (IGF-I), KC4-antigen, KS-1-antigen, KS1-4, Le-Y, macrophage inhibition factor (MIF), MAGE, MUC1, MUC2, MUC3, MUC4, NCA66, NCA95, NCA90, tyrosinase, PRAME, EBNA, KLK3, HPV E7, LMP2, NY-ESO-1, PAP, reverse transcriptase, nucleophosmin, PRTN3/ELANE, CT83/KKLC1, MUC16, DNTT, antigen specific for PAM-4 antibody, placental growth factor, p53, prostatic acid phosphatase, RS5, S1OO, TAC, TAG-72, tenascin, TRAIL receptors, Tn antigen, Thomson-Friedenreich antigens, tumor necrosis antigens, VEGF, ED-B fibronectin, 17-1A-antigen, NeuGcGM3, N-glycolyl GM3 ganglioside, Neu5Gc, GM3-Ganglioside, GD3, GM2, carbohydrate antigens, ganglioside antigens, Lewis Y, Lewis B, MOG, MBP, aB-crystallin, PLP, GlialCAM, β-synuclein, HLA-A2, TNP, CD123, Kappa chain of immunoglobulin, or any combination thereof.

15. The CAR polypeptide according to any one of claims 12 to 14, wherein the transmembrane domain is selected from the group comprising CD28, CD28T, OX-40, 4-1BB/CD137, CD2, CD7, CD27, CD30, CD40, programmed death-1 (PD-1), inducible T cell costimulator (ICOS), CDl-la/CD18, CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, or any combination thereof.

16. The CAR polypeptide according to any one of claims 12 to 15, wherein the costimulatory domain is a signalling region (or other suitable portion) comprising CD28, OX-40, 4-1BB/CD137, CD2, CD7, CD27, CD30, CD40, programmed death-1 (PD-1), inducible T cell costimulator (ICOS), lymphocyte function-associated antigen-1 (LFA-1 (CDl la/CD18), CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class I molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD 19a, a ligand that specifically binds with CD83, or any combination thereof.

17. The CAR polypeptide according to any one of claims 12 to 16, wherein the at least one activating domain is a T cell activation domain, preferably a CD3 domain, more preferably a CD3 zeta (CD3ζ) domain.

18. The CAR polypeptide according to any one of claims 12 to 17, further comprises a self-cleaving domain comprised between the at least one activating domain, and the at least one interleukin 4 (IL-4) molecule, wherein the self-cleaving domain comprises the sequence DxExNPGP (wherein x is any amino-acid).
